(19) Europäisches Patentamt
European Patent Office
Office européen des brevets

(11) Veröffentlichungsnummer: **0 111 447**
**B1**

(12) # EUROPÄISCHE PATENTSCHRIFT

(45) Veröffentlichungstag der Patentschrift: **07.01.88**

(21) Anmeldenummer: **83810554.2**

(22) Anmeldetag: **28.11.83**

(51) Int. Cl.⁴: **G 03 C 7/26,** C 07 D 471/10, C 07 D 491/113 // (C07D471/10, 235:00, 221:00),(C07D491/113, 317:00, 221:00)

(54) **Farbphotographisches Aufzeichnungsmaterial.**

(30) Priorität: **03.12.82 CH 7046/82**

(43) Veröffentlichungstag der Anmeldung: **20.06.84 Patentblatt 84/25**

(45) Bekanntmachung des Hinweises auf die Patenterteilung: **07.01.88 Patentblatt 88/1**

(84) Benannte Vertragsstaaten: **BE DE FR GB IT**

(56) Entgegenhaltungen:
**EP-A-0 011 051**
**DE-A-2 126 187**
**FR-A-2 334 672**
**US-A-4 237 294**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Leppard, David G., Dr., Route de Bourguillon 6, CH-1723 Marly (CH)**
Erfinder: **Rody, Jean, Dr., Rütiring 82, CH-4125 Riehen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Anmeldung betrifft ein farbphotographisches Aufzeichnungsmaterial, das in mindestens einer lichtempfindlichen Silberhalogenidemulsionsschicht und/oder in mindestens einer der üblichen Hilfsschichten als Stabilisator eine spezifische Polyalkylpiperidinspiroverbindung enthält.

Polyalkylpiperidine sind als sterisch gehinderte Amine allgemein als Lichtschutzmittel für organische Materialien, insbesondere für Polymere, bekannt. Es wurde auch bereits in der DE-A-2 126 187 vorgeschlagen, solche Polyalkylpiperidine als Mittel gegen das Ausbleichen von Farbphotographien zu verwenden. Es wurde weiterhin in der EP-A-0 011 051 vorgeschlagen, als Lichtschutzmittel für Farbphotographien bestimmte Polyalkylpiperidinderivate zu verwenden, die mindestens eine Phenolgruppe enthalten. Es handelt sich dabei um Polyalkylpiperidinester von Hydroxylbenzylmalonsäuren.

In Weiterführung dieser Forschungsarbeiten wurde gefunden, dass Verbindungen, die in ihrem Molekül mindestens eine sterisch gehinderte Phenolgruppe und mindestens eine Polyalkylpiperidinspirogruppe enthalten eine überraschend bessere stabilisierende Wirkung entfalten.

Gegenstand der vorliegenden Erfindung ist daher ein farbphotographisches Aufzeichnungsmaterial, das in mindestens einer lichtempfindlichen Silberhalogenidemulsionsschicht, einer Zwischenschicht und/oder einer Schutzschicht mindestens eine Polyalkylpiperidinverbindung als Stabilisator enthält, dadurch gekennzeichnet, dass die Polyalkylpiperidinverbindung einer der Formeln I oder II

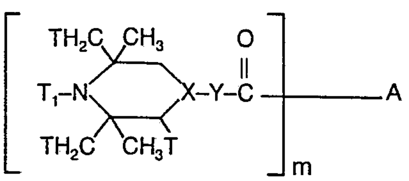 (I)

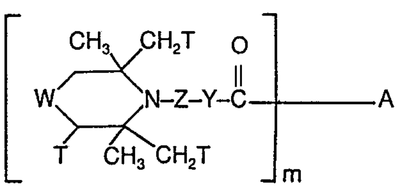 (II)

entspricht, worin

m die Zahlen 1 oder 2 bedeutet,

A bei m = 1 eine Gruppe der Formel

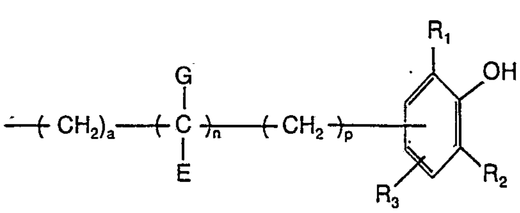

und bei m = 2 eine Gruppe der Formel

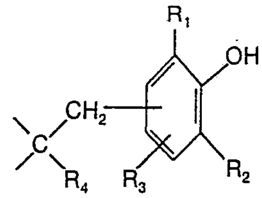

ist, worin

$R_1$ Wasserstoff, $C_1$–$C_{12}$ Alkyl, $C_5$–$C_8$ Cycloalkyl, $C_7$–$C_9$ Phenylalkyl, Phenyl oder $C_7$–$C_{10}$ Alkylphenyl bedeutet,

$R_2$ $C_1$–$C_8$ Alkyl, $C_5$–$C_8$ Cycloalkyl, $C_7$–$C_9$ Phenylalkyl, Phenyl oder $C_7$–$C_{10}$ Alkylphenyl ist,

$R_3$ Wasserstoff oder Methyl ist

$R_4$ Wasserstoff, $C_1$–$C_{12}$ Alkyl, Allyl, Benzyl, Cyclohexyl oder eine Gruppe

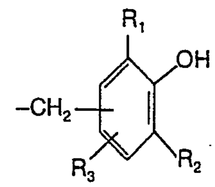 ist,

G Wasserstoff oder eine Gruppe

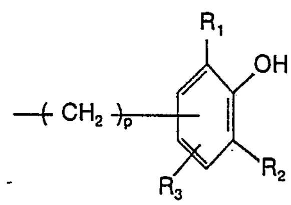

bedeutet,

E Wasserstoff, Methyl, –CN, $-COR_5$ oder $-COOR_5$ ist, wobei $R_5$ $C_1$–$C_8$ Alkyl oder $C_3$–$C_4$ Alkoxyalkyl bedeutet und

n und p unabhängig voneinander die Zahlen 0 oder 1 und a die Zahlen 0, 1 oder 2 bedeuten,

Y –O– oder $-N(R_6)-$ ist, wobei $R_6$ Wasserstoff, $C_1$–$C_{18}$ Alkyl, $C_3$–$C_{12}$ Alkenyl, $C_5$–$C_8$ Cycloalkyl, Phenyl, $C_7$–$C_{14}$ Alkaryl, $C_7$–$C_{14}$ Aralkyl oder $C_3$–$C_4$ Alkoxyalkyl ist

Z eine Gruppe 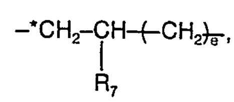

worin $R_7$ Wasserstoff, Methyl, Ethyl, Phenoxymethyl, Phenyl oder $OR_8$ ist, $-OR_8$ aber nur dann, wenn e = 1 ist, und worin $R_8$ Wasserstoff oder –COL bedeutet, L $C_1$–$C_4$-Alkyl ist, e die Zahl 0 oder 1 ist und das *C am Piperidinstickstoff gebunden ist, bedeutet,

T Wasserstoff oder Methyl ist,

$T_1$ Hydroxy, $C_1$–$C_{12}$ Alkyl, $C_3$–$C_6$ Alkenylmethyl oder $C_3$–$C_4$ Alkinylmethyl, sowie $C_7$–$C_{14}$ Aralkyl, Glycidyl, durch Halogen, Cyano, $-COOR_9$ oder $-CON(R_{10})(R_{11})$ substituiertes $C_1$–$C_4$-Alkyl, eine Gruppe $-COR_{12}$, $-COOR_9$ oder $-CON(R_{10})(R_{11})$, eine Gruppe $-CH_2-CH(R_{13})-OR_{14}$, $-SOR_{15}$, $-SO_2R_{15}$, $-OR_9$, $-OOCR_{12}$ ist, wobei $R_9$ $C_1$–$C_{12}$ Alkyl, Allyl, Cyclohexyl oder Benzyl, $R_{10}$ $C_1$–$C_{12}$ Alkyl, Allyl, Cyclohexyl, Benzyl, Phenyl oder $C_7$–$C_{10}$ Alkylphenyl, $R_{11}$ Wasserstoff, $C_1$–$C_{12}$-Alkyl, Allyl, Cyclohexyl oder Benzyl sind oder $R_{10}$ und $R_{11}$ zusammen mit dem N-Atom, an das sie gebunden sind,

einen 5- oder 6-gliedrigen heterocyclischen Ring bilden, $R_{12}$ Wasserstoff, $C_1$–$C_{12}$ Alkyl, $C_2$–$C_6$ Alkenyl, Chlormethyl, $C_5$–$C_8$ Cycloalkyl, $C_7$–$C_{14}$ Aralkyl, Phenyl, $C_7$–$C_{10}$ Alkylphenyl oder durch 1 oder 2 $C_1$–$C_4$ Alkyl und 1 Hydroxyl substituiertes Phenyl, Phenylmethyl oder Phenylethyl, $R_{13}$ Wasserstoff, $C_1$–$C_4$ Alkyl, $C_3$–$C_4$ Alkoxyalkyl, Phenyl oder Phenoxymethyl, $R_{14}$ Wasserstoff, $C_1$–$C_{12}$ Alkyl, eine Gruppe $-COR_{12}$ oder $-CON(R_{10})(R_{11})$ und $R_{15}$ $C_1$–$C_{12}$ Alkyl, Phenyl oder $C_7$–$C_{10}$ Alkylphenyl bedeuten, oder $T_1$ eine Gruppe der Formel

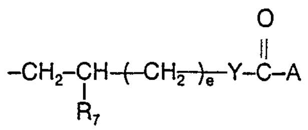

oder der Formel

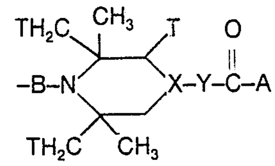

bedeutet, worin B eine Gruppe $C_rH_{2r}$ ist, in welcher r eine Zahl 2 bis 12 bedeutet, oder $C_4$–$C_8$ Alkenylen, $C_4$–$C_8$ Alinylen, Phenylen, Xylylen, Bitolylen, $C_5$–$C_{12}$ Cycloalkylen oder eine Gruppe $-CONH-B_1-NHCO-$, worin $B_1$ eine Gruppe $C_rH_{2r}$, Phenylen, Naphthylen, Tolylen oder eine Gruppe der Formeln

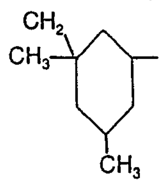 , 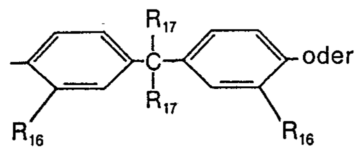 oder 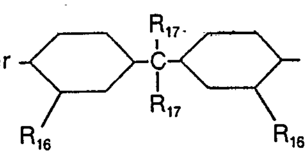

bedeutet, worin $R_{16}$ Wasserstoff oder Methyl, $R_{17}$ Wasserstoff, Methyl oder Ethyl sind, X eine der Gruppen

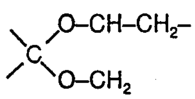

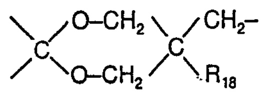

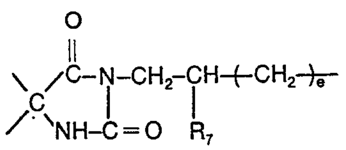

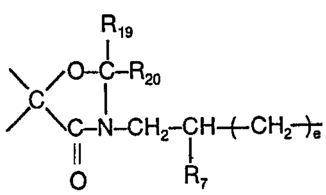

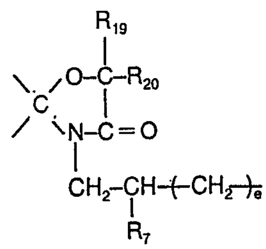

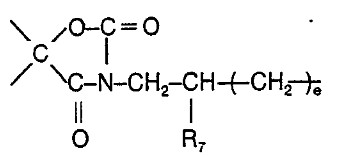

und

W eine der Gruppen

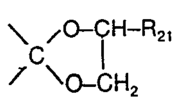

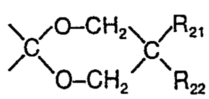

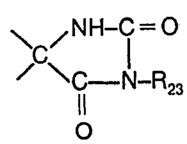

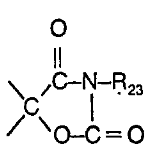

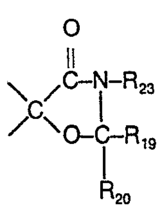

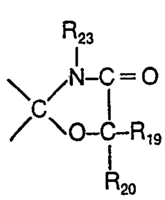

worin

$R_{18}$ Methyl oder Ethyl,

$R_{19}$ Wasserstoff, $C_1$–$C_{12}$ Alkyl, $C_5$–$C_8$ Cycloalkyl oder $C_7$–$C_{14}$ Aralkyl,

$R_{20}$ $C_1$–$C_{12}$ Alkyl, $C_5$–$C_8$ Cycloalkyl oder Phenyl sind oder

$R_{19}$ und $R_{20}$ zusammen mit dem C-Atom, an das sie gebunden sind, einen $C_5$–$C_{12}$ Cycloalkan- oder Alkylcycloalkanring bilden,

$R_{21}$ Wasserstoff, $C_1$–$C_{12}$ Alkyl, eine Gruppe der Formel $-CH_2-OCOR_{24}$ ist, worin $R_{24}$ Wasserstoff, $C_1$–$C_4$ Alkyl, $C_2$–$C_6$ Alkenyl, Cyclohexyl, Phenyl, Benzyl oder Chlormethyl oder eine Gruppe $-CH_2-O-SO_2R_{27}$, worin $R_{27}$ Methyl, Phenyl oder p-Tolyl ist, bedeutet, oder eine der Gruppen

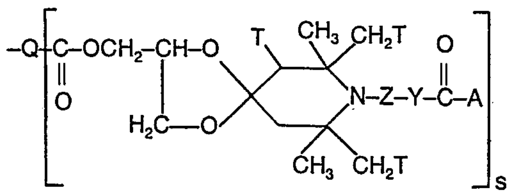

oder

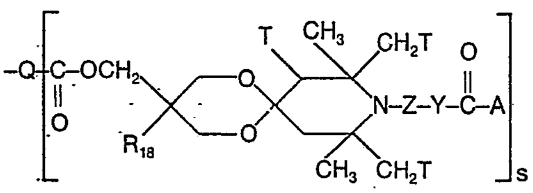

ist worin s die Zahl 1, 2 oder 3 bedeutet und

Q bei s = 1 die oben für B angegebene Bedeutung hat bei s = 2 ein dreiwertiger Rest der Formeln

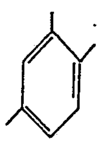

oder

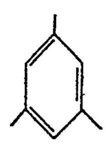

ist und

bei s = 3 ein vierwertiger Rest der Formeln

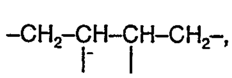,

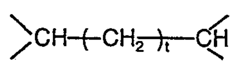

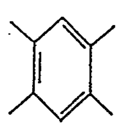

oder

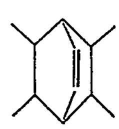

ist, wobei t

die Zahlen 1 bis 8 bedeutet, und

$R_{21}$ zusätzlich auch noch eine Gruppe der Formel $-CH_2O-SO R_{25}$, worin $R_{25}$ $C_1$–$C_4$ Alkyl, p-Tolyl oder Phenyl ist, eine Gruppe der Formel $-CH_2-CO-NHR_{26}$, worin $R_{26}$ Wasserstoff oder $C_1$–$C_4$ Alkyl ist,

$R_{22}$ Wasserstoff oder $C_1$–$C_4$ Alkyl und

$R_{23}$ Wasserstoff, $C_1$–$C_{12}$ Alkyl, $C_3$–$C_4$ Alkoxyalkyl, $C_5$–$C_8$ Cycloalkyl, Allyl oder Benzyl bedeuten, und, falls m = 1 ist, W zusätzlich auch eine Gruppe der Formel

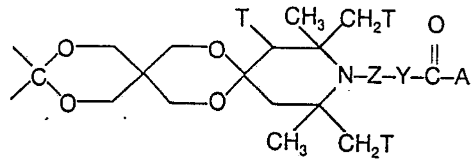

sein kann, wobei die wiederholt erwähnten Reste und Symbole immer die erstmals angegebene Bedeutung haben.

Bedeuten etwaige Substituenten Alkyl, so handelt es sich um geradkettige oder verzweigte Alkylgruppen. Bedeuten sie $C_1$–$C_4$ Alkyl, dann handelt es sich um Methyl, Ethyl, n-Propyl, Isopropyl, n-Butyl, sec.-Butyl oder tert.-Butyl. Bedeuten sie $C_1$–$C_8$ Alkyl, so kommen zusätzlich z.B. n-Pentyl, 2,2-Dimethylpropyl, n-Hexyl, 2,3-Dimethylbutyl, n-Octyl oder 1,1,3,3-Tetramethylbutyl in Frage. Bedeuten sie $C_1$–$C_{12}$ Alkyl, so können sie zusätzlich auch z.B. Nonyl, Decyl, Undecyl und Dodecyl sein. $R_6$ bedeutet als $C_1$–$C_{18}$ Alkyl zusätzlich z.B. Tetradecyl, Hexadecyl, Heptadecyl oder Octadecyl.

$T_1$ kann als substituiertes $C_1$–$C_4$ Alkyl beispielsweise substituiertes Methyl, in 1- oder 2-Position substituiertes Ethyl, in 1-, 2- oder 3-Position substituiertes Propyl oder in 1-, 2-, 3- oder 4-Position substituiertes n-Butyl bedeuten. Es kann sich auch um substituiertes tert.-Butyl oder sek.-Butyl handeln.

Bedeuten etwaige Substituenten $C_5$–$C_8$ Cycloalkyl, so handelt es sich z.B. um Cyclopentyl, Cyclohexyl, Cycloheptyl, α-Methylcyclohexyl, Cyclooctyl oder Dimethylcyclohexyl.

Bilden $R_{19}$ und $R_{20}$ zusammen mit dem C-Atom, an das sie gebunden sind, einen $C_5$–$C_{12}$ Cycloalkan- oder Alkylcycloalkanring, so handelt es sich beispielsweise um Cyclopentan, Cyclohexan, Cycloheptan, Cyclooctan, Cyclodecan, Cyclododecan, Methylcyclohexan oder Dimethylcyclohexan.

$R_1$ und $R_2$ bedeuten als $C_7$–$C_9$ Phenylalkyl beispielsweise Benzyl, Phenylethyl oder Phenylpropyl. Bedeuten $T_1$, $R_6$, $R_{12}$ und $R_{19}$ $C_7$–$C_{14}$ Aralkyl, so handelt es sich darüber hinaus auch z.B. um Phenylbutyl oder Naphthylmethyl.

$R_1$, $R_2$, $R_{10}$, $R_{12}$ und $R_{15}$ können als $C_7$–$C_{10}$ Alkylphenyl beispielsweise Tolyl, Xylyl, Isopropylphenyl, tert.-Butylphenyl oder Diethylphenyl sein.

$R_6$, $R_{12}$ und $R_{24}$ als $C_3$–$C_6$ Alkenyl, sowie $T_1$ als $C_3$–$C_6$ Alkenylmethyl, sind z.B. Allyl, Methallyl, Dimethylallyl oder 2-Hexenyl, $R_{12}$ und $R_{24}$ können als $C_2$–$C_6$ Alkenyl zusätzlich auch Vinyl sein.

Als $C_3$–$C_{12}$ Alkenyl kann $R_6$ zusätzlich auch 2-Octenyl, 2-Decenyl oder 2-Dodecenyl sein.

$T_1$ bedeutet als $C_3$–$C_4$ Alkinylmethyl z.B. Propargyl, n-But-4-inyl oder n-But-3-inyl. Bevorzugt ist Propargyl.

Bedeutet $R_6$ $C_7$–$C_{14}$ Alkaryl, so kann es sein: durch $C_1$–$C_4$ Alkyl substituiertes Phenyl, wie p-Tolyl, 2,4-Dimethylphenyl, 2,6-Dimethylphenyl, 2,4-Diethylphenyl, 2,6-Diethylphenyl, 4-tert.-Butylphenyl, 2,4-Di-tert-butylphenyl, oder 2,6-Di-tert.-butylphenyl, Bevorzugt sind 2,4-Di-tert.-butylphenyl und 2,4-Dimethylphenyl.

$R_5$, $R_6$, $R_{13}$ und $R_{23}$ bedeuten als $C_3$–$C_4$ Alkoxyalkyl z.B. Ethoxymethyl, 2-Methoxyethyl oder 2-Ethoxyethyl.

Ist B $C_4$–$C_8$ Alkenylen, so handelt es sich z.B. um 2-Butenylen-1,4.

Ist B $C_4$–$C_8$ Alkinylen, so handelt es sich z.B. um 2-Butinylen-1,4.

Bedeutet B $C_5$–$C_{12}$ Cycloalkylen, so ist es z.B. Cyclopentylen, Cyclohexylen, Cyclooctylen, Cyclodecylen oder Cyclododecylen. Bevorzugt ist Cyclohexylen.

$R_{12}$ bedeutet als durch 1 oder 2 $C_1$–$C_4$ Alkyl und 1-Hydroxyl substituiertes Phenyl, Phenylmethyl oder Phenylethyl, z.B. 3,5-Dimethyl-4-hydroxyphenyl, 3,5-Di-tert.butyl-4-hydroxyphenyl, 3,5-Dimethyl-4-hydroxyphenyl, 3,5-Dimethyl-4-hydroxybenzyl, 3,5-Di-tert.butyl-4-hydroxybenzyl oder 2-(3,5-Di-tert.-butyl-4-hydroxyphenyl)-ethyl.

Bedeuten etwaige Substituenten Halogen, so handelt es sich z.B. um Brom, Iod und insbesondere Chlor.

Besonders interessant sind als Stabilisatoren Verbindungen der Formeln III und IV

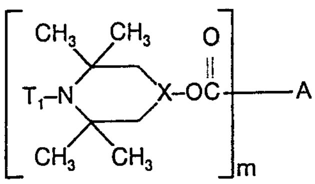

(III)

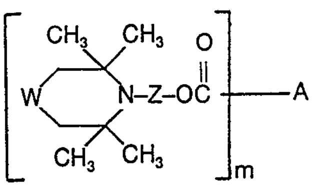

(IV)

worin

m die Zahlen 1 oder 2 bedeutet

A bei m = 1 eine Gruppe der Formel

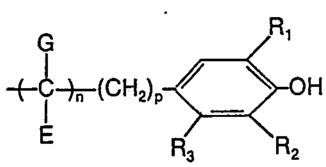

und bei m = 2 eine Gruppe der Formel

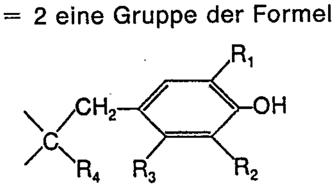

ist, worin

$R_1$ $C_1$–$C_4$ Alkyl,

$R_2$ Wasserstoff oder $C_1$–$C_4$ Alkyl,

$R_3$ Wasserstoff oder Methyl,

$R_4$ Wasserstoff, $C_1$–$C_4$ Alkyl oder eine Gruppe

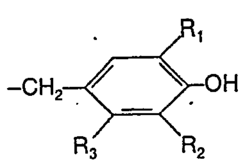

G Wasserstoff oder eine Gruppe

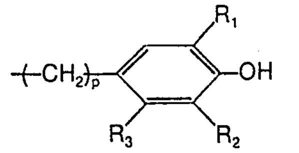

E Wasserstoff, Methyl, –CN oder $-COCH_3$ und

n und p unabhängig voneinander die Zahlen 0 oder 1 bedeuten,

Z eine Gruppe 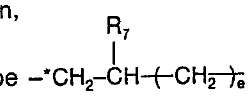

ist, worin

$R_7$ Wasserstoff, Methyl, Ethyl, Phenoxymethyl und Phenyl ist, e die Zahlen 0 oder 1 und das *C am Piperidinstickstoff gebunden ist,

$T_1$ Hydroxy, $C_1$–$C_4$ Alkoxy, Acetoxy, $C_1$–$C_4$ Alkyl, $C_3$–$C_4$ Alkenylmethyl, Propargyl, Glycidyl, Benzyl, durch $-COOR_9$ substituiertes Methyl oder Ethyl, eine Gruppe $-COR_{12}$, $-COOR_9$ oder $-CON(R_{10})(R_{11})$, eine Gruppe $-CH_2-CH(R_{13})-OR_{14}$ ist, wobei $R_9$ $C_1$–$C_8$ Alkyl, Allyl oder Cyclohexyl, $R_{10}$ $C_1$–$C_{12}$ Alkyl, Cyclohexyl oder Phenyl, $R_{11}$ Wasserstoff oder $C_1$–$C_{12}$ Alkyl sind oder $R_{10}$ und $R_{11}$ zusammen mit dem N-Atom, an das sie gebunden sind, einen 6-gliedrigen heterocyclischen Ring bilden, $R_{12}$ $C_1$–$C_{12}$ Alkyl, $C_2$–$C_4$ Alkenyl, Cyclohexyl, Benzyl, Phenyl oder 2-(3,5-Di-tert.-butyl-4-hydroxy-phenyl)-ethyl, $R_{13}$ Wasserstoff, Methyl oder Phenyl und $R_{14}$ Wasserstoff, $C_1$–$C_4$ Alkyl, eine Gruppe $-COR_{12}$ oder $-CON(R_{10})(R_{11})$ bedeuten oder

$T_1$ eine Gruppe der Formel

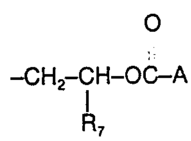

oder der Formel

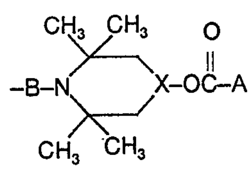

bedeutet, worin B eine Gruppe $-(-CH_2-)_r-$ oder $-CONH-(-CH_2-)_r-NHCO-$, worin r die Zahlen 2 bis 8 bedeutet, oder $C_4$–$C_8$ Alkenylen, Xylylen oder Bitolylen ist,

X eine der Gruppen

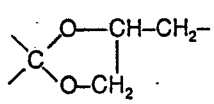

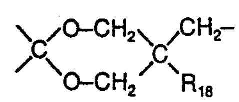

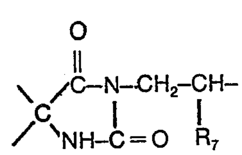

und

W eine der Gruppen

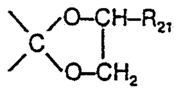

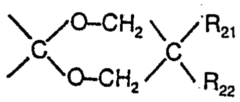

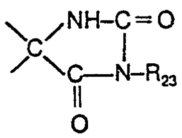

worin

$R_{18}$ Methyl oder Ethyl bedeutet

$R_{21}$ Wasserstoff-, $C_1$–$C_8$ Alkyl, eine Gruppe der Formel $-CH_2-OCOR_{24}$, worin $R_{24}$ $C_1$–$C_4$ Alkyl, Allyl, Phenyl oder Benzyl ist, oder eine Gruppe der Formel $-CH_2O-SO_2R_{25}$, worin $R_{25}$ Methyl, Phenyl oder p.Tolyl ist, bedeutet,

$R_{22}$ Wasserstoff, Methyl oder Ethyl ist und

$R_{23}$ Wasserstoff, $C_1$–$C_8$ Alkyl, Cyclohexyl, Allyl oder Benzyl bedeutet und, falls m = 1 ist, W zusätzlich auch eine Gruppe der Formel

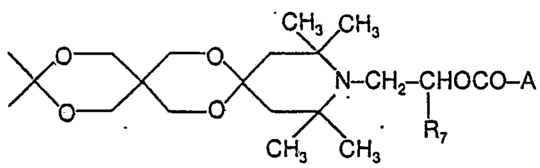

sein kann, wobei die in dieser Bevorzugung wiederholt erwähnten Reste und Symbole immer die in dieser Bevorzugung erstmals angegebene Bedeutung haben.

Bevorzugt sind als Stabilisatoren Verbindungen der Formel III, worin

m die Zahlen 1 oder 2 bedeutet,

A bei m = 1 eine Gruppe der Formel

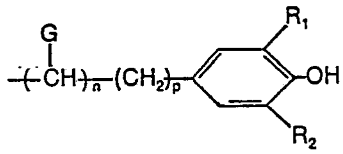

und bei m = 2 eine Gruppe der Formel

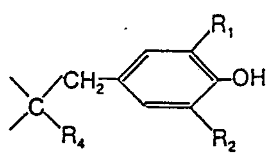

ist, worin

$R_1$ Wasserstoff, Methyl oder tert.-Butyl,

$R_2$ Methyl oder tert.-Butyl,

$R_4$ Wasserstoff, $C_1$–$C_4$ Alkyl oder eine Gruppe

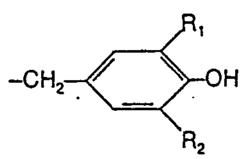

G Wasserstoff oder eine Gruppe

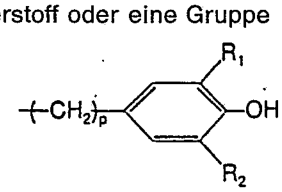

n und p unabhängig voneinander die Zahlen 0 oder 1 bedeuten,

$T_1$ Methoxy, Methyl, Allyl, Benzyl, Acetyl, Acryloyl oder eine Gruppe $-CON(R_{10})(R_{11})$ ist, wobei $R_{10}$ $C_1$–$C_4$ Alkyl, Cyclohexyl oder Phenyl $R_{11}$ Wasserstoff oder $C_1$–$C_4$ Alkyl bedeuten oder

$T_1$ eine Gruppe $-CH_2CH_2-OCO-A$ ist,

X eine der Gruppen

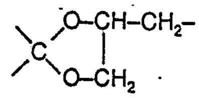

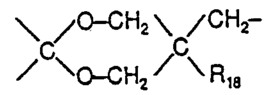

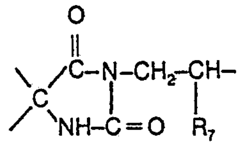

worin $R_7$ Wasserstoff, Methyl oder Phenyl und $R_{18}$ Methyl oder Ethyl ist, bedeutet, wobei die in dieser Bevorzugung wiederholt erwähnten Reste immer die in dieser Bevorzugung erstmals angegebene Bedeutung haben. Ebenfalls bevorzugt als Stabilisatoren sind Verbindungen der Formel IV, worin

m die Zahl 1 oder 2 bedeutet,

A bei m = 1 eine Gruppe der Formel

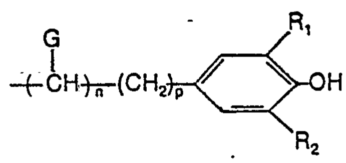

und bei m = 2 eine Gruppe der Formel

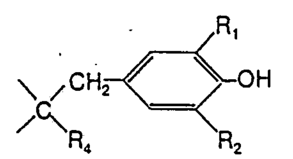

ist, worin

$R_1$ Wasserstoff, Methyl oder tert.-Butyl,

$R_2$ Methyl oder tert.-Butyl,

$R_4$ Wasserstoff, $C_1$–$C_4$ Alkyl oder eine Gruppe

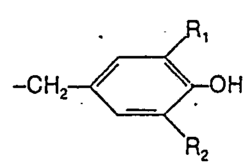

G Wasserstoff oder eine Gruppe

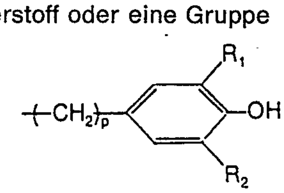

n und p unabhängig voneinander die Zahlen 0 oder 1 bedeuten,

Z eine Gruppe 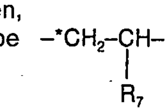

ist, worin $R_7$

Wasserstoff, Methyl oder Phenyl ist und das *C am Piperidinstickstoff gebunden ist,

$T_1$ Methoxy, Methyl, Allyl, Benzyl, Acetyl, Acryloyl oder eine Gruppe $-CON(R_{10})(R_{11})$, $-SOR_{15}$ oder $-SO_2R_{15}$ ist, wobei $R_{10}$ $C_1$–$C_4$ Alkyl, Cyclohexyl oder Phenyl, $R_{11}$ Wasserstoff oder $C_1$–$C_4$ Alkyl und $R_{15}$ Methyl, Phenyl oder p-Tolyl bedeuten oder

$T_1$ eine Gruppe $-CH_2CH_2-OCO-A$ ist,

W eine der Gruppen

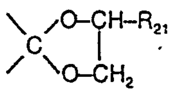

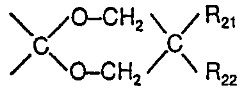

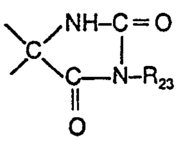

worin

$R_{21}$ Wasserstoff, $C_1$–$C_8$ Alkyl, eine Gruppe der Formel $-CH_2-OCOR_{24}$, worin $R_{24}$ $C_1$–$C_4$ Alkyl, Allyl oder Benzyl ist oder eine Gruppe der Formel $-CH_2O-SO_2R_{27}$, worin $R_{27}$ Methyl, Phenyl oder p-Tolyl ist, bedeutet,

$R_{22}$ Wasserstoff, Methyl oder Ethyl ist und

$R_{23}$ Wasserstoff, $C_1$–$C_8$ Alkyl, Allyl oder Benzyl bedeutet, und, falls m = 1 ist, W zusätzlich auch eine Gruppe der Formel

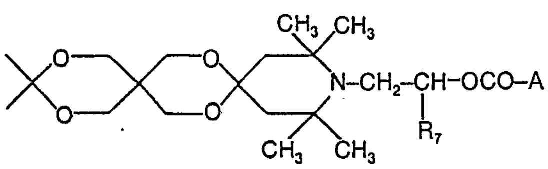

sein kann, wobei die in dieser Bevorzugung wiederholt erwähnten Reste immer die in dieser Bevorzugung erstmals angegebene Bedeutung haben.

Bei den Verbindungen der Formeln I und II handelt es sich um Spiropiperidine mit phenolischen Substituenten. Solche sind z.B. bekannt aus der US-A-4 237 294 und werden dort als Stabilisatoren für synthetische Polymere empfohlen.

Ähnliche Spiropiperidine – mit oder ohne phenolische Substituenten – sind beschrieben in den US-A-3 705 126, 3 790 525, 3 859 293, 3 941 744 und den DE-A-2 606 026 und 2 634 957.

Soweit die vorhin beschriebenen Verbindungen noch neu sind, können sie in Analogie zu den bekannten Verbindungen gemäss den oben angegebenen Publikationen hergestellt werden. Die letzte Stufe der Synthese ist dabei meist eine Veresterung (aus Säure + Alkohol oder Säurechlorid + Alkohol) oder eine Umesterung oder eine Amidierung.

Typische Vertreter von Verbindungen der Formel I sind in der nachstehenden Tabelle I und von Verbindungen der Formel II in der nachstehenden Tabelle II aufgeführt.

Tabelle 1

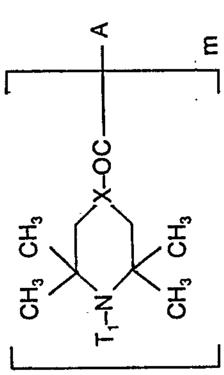

| Stabilisator Nr. | $T_1$ | X | m | A |
|---|---|---|---|---|
| 1 | $CH_3CO-$ | 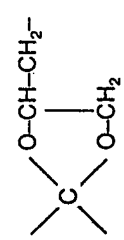 | 1 | 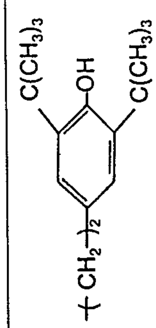 |
| 2 | $CH_3-$ | 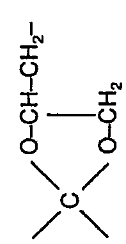 | 1 | 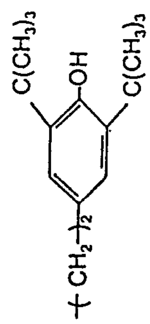 |

Tabelle 1 (Fortsetzung)

| Stabilisator Nr. | $T_1$ | X | m | A |
|---|---|---|---|---|
| 3 | $CH_2=CH-CO-$ | 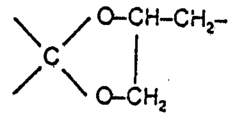 | 1 | 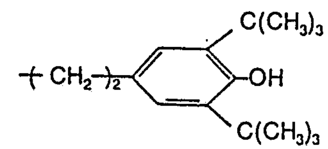 |
| 4 | $CH_3CO-$ | 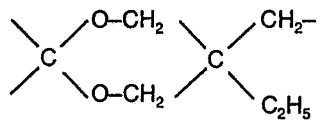 | 1 | 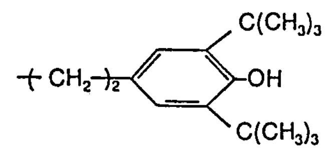 |
| 5 | $CH_2=CH-CO-$ | 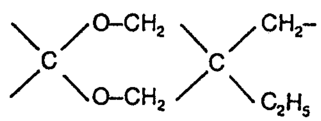 | 1 | 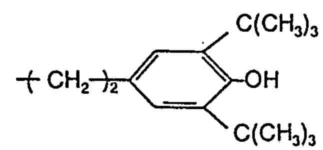 |
| 6 | $CH_2=CH-CH_2-$ | 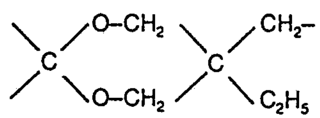 | 1 | 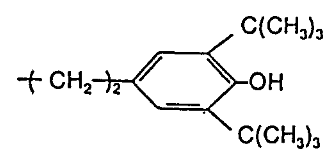 |
| 7 | $CH_2=CH-CO-$ | 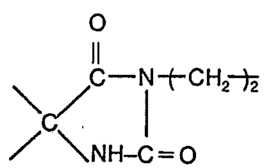 | 1 | 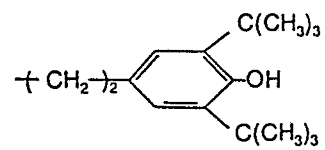 |
| 8 | $CH_2=CH-CO-$ | 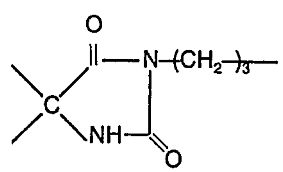 | 1 | 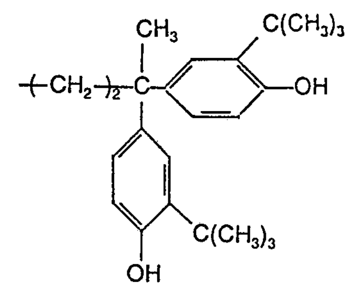 |

Tabelle 1 (Fortsetzung)

| Stabilisator Nr. | $T_1$ | X | m | A |
|---|---|---|---|---|
| 9 | $CH_2=CH-CO-$ | 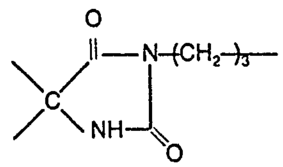 | 1 | 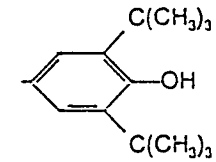 |
| 10 | $CH_3CO-$ | 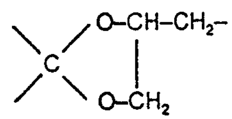 | 1 | 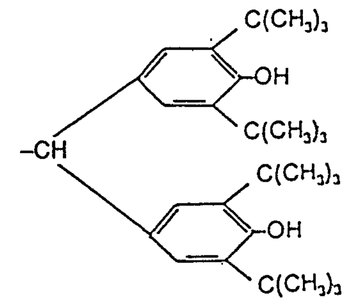 |
| 11 | $(C_2H_5)_2NCO-$ | 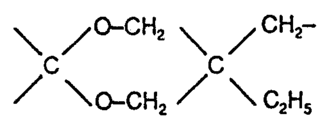 | 1 | 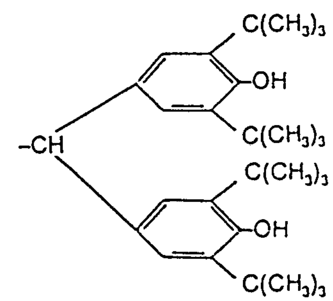 |
| 12 | 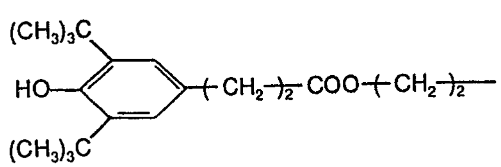 | 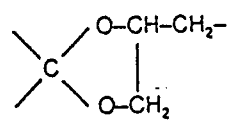 | 1 | 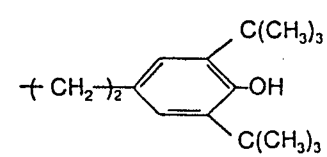 |

Tabelle 1 (Fortsetzung)

| Stabilisator Nr. | $T_1$ | X | m | A |
|---|---|---|---|---|
| 13 | $(C_2H_5)_2-NCO-$ | 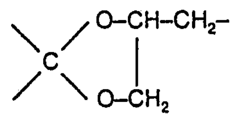 | 1 | 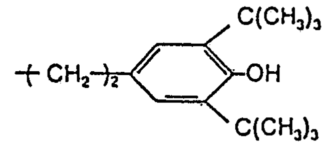 |
| 14 | $CH_3$ | 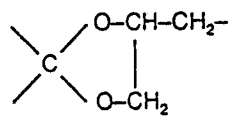 | 1 | 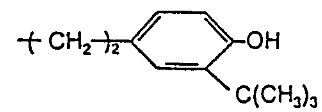 |
| 15 | 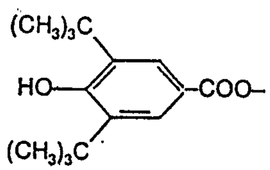 | 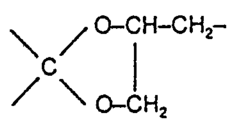 | 1 | 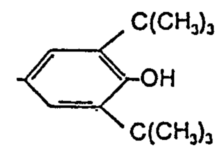 |
| 16 | 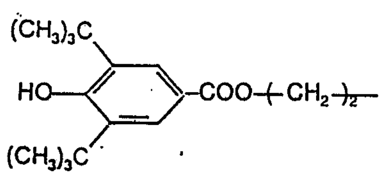 | 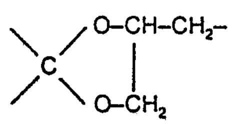 | 1 | 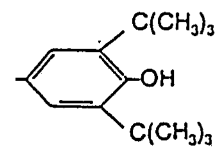 |
| 17 | $CH_3CO-$ | 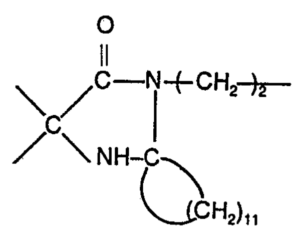 | 1 | 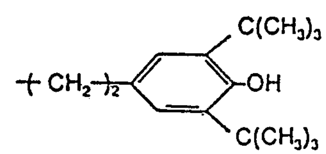 |

Tabelle 1 (Fortsetzung)

| Stabilisator Nr. | $T_1$ | X | m | A |
|---|---|---|---|---|
| 18 | HO– | 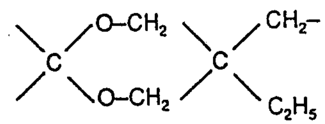 | 1 | 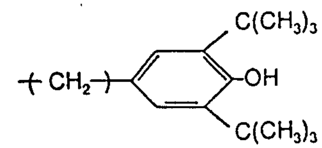 |
| 19 | $CH_3$– | 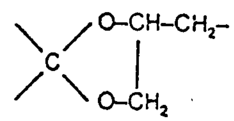 | 1 | 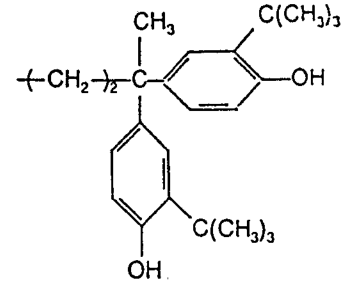 |
| 20 | 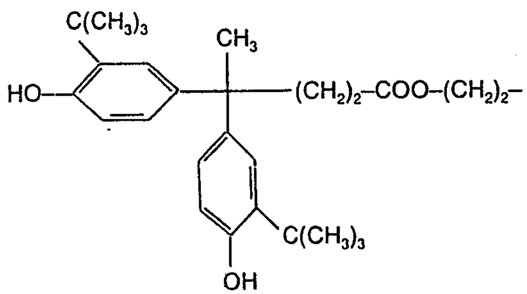 | 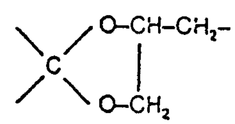 | 1 | 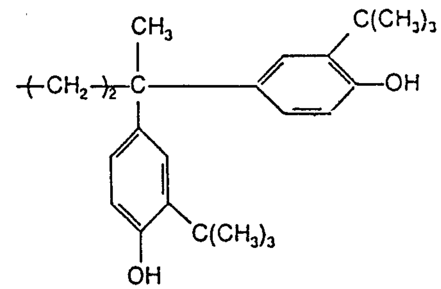 |
| 21 | 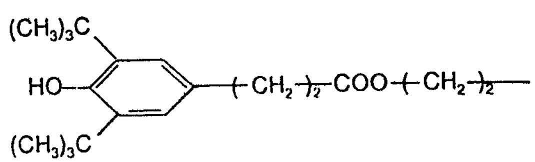 | 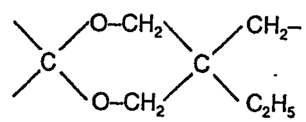 | 1 | 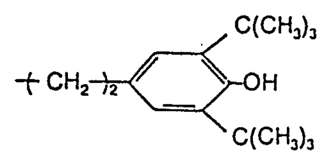 |

Tabelle 1 (Fortsetzung)

| Stabilisator Nr. | $T_1$ | X | m | A |
|---|---|---|---|---|
| 22 | 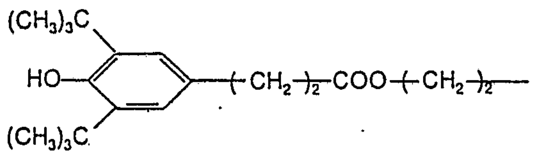 | 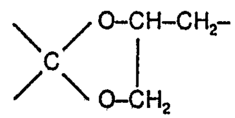 | 1 | 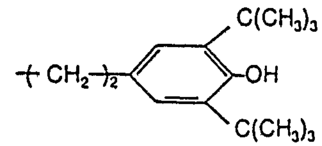 |
| 23 | $CH_3CO-$ | 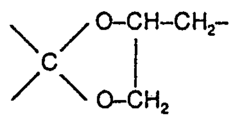 | 2 | 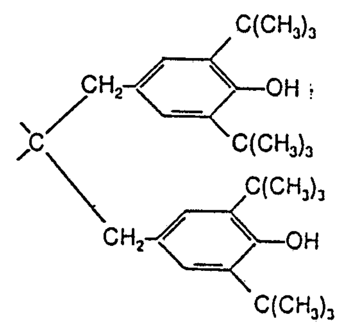 |
| 24 | $CH_3CO-$ | 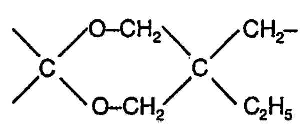 | 2 | 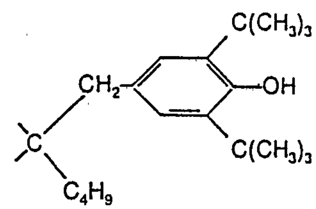 |
| 25 | $CH_3-$ | 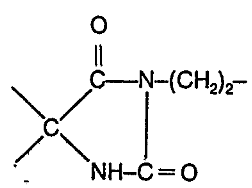 | 2 | 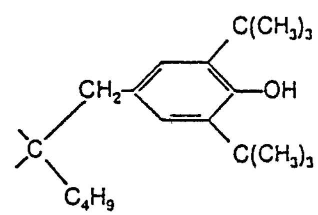 |

Tabelle 1 (Fortsetzung)

| Stabilisator Nr. | $T_1$ | X | m | A |
|---|---|---|---|---|
| 26 | $CH_3-$ | 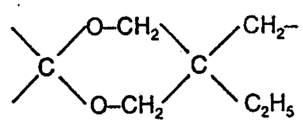 | 2 | 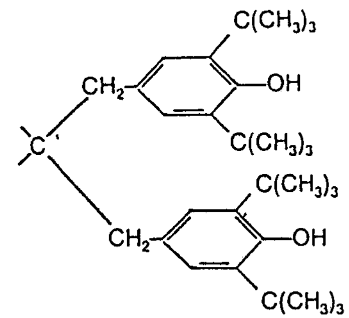 |
| 27 | $CH_3-$ | 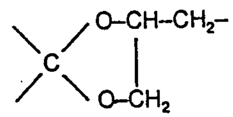 | 2 | 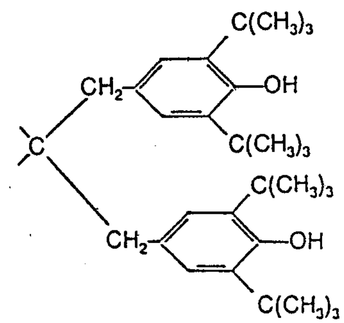 |
| 28 | $CH_3CO-$ | 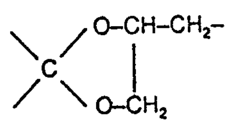 | 2 | 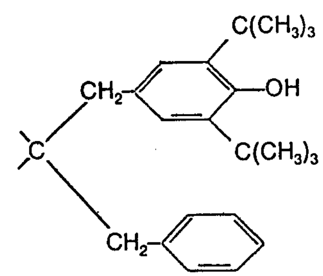 |

Tabelle II

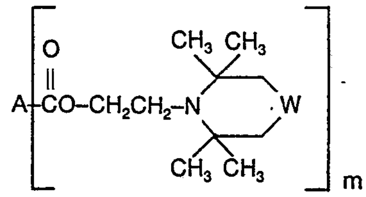

| Stabilisator Nr. | A | m | W |
|---|---|---|---|
| 29 | 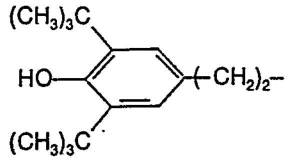 | 1 | 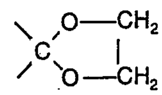 |
| 30 | 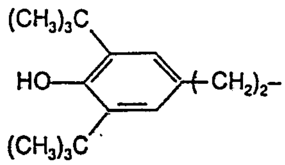 | 1 | 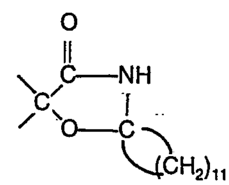 |
| 31 | 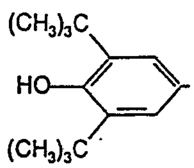 | 1 | 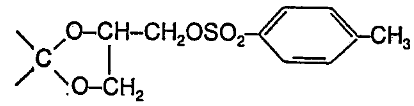 |
| 32 | 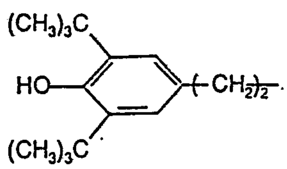 | 1 | 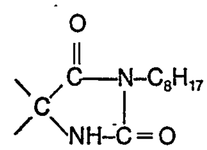 |
| 33 | 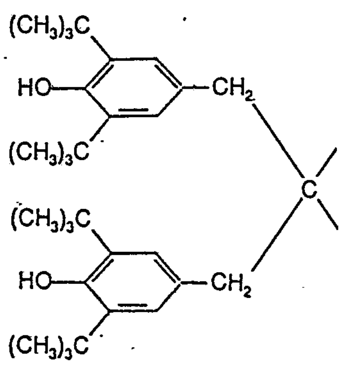 | 2 | 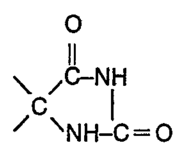 |

Weitere typische Vertreter von Verbindungen der Formel II sind die Stabilisatoren.

34

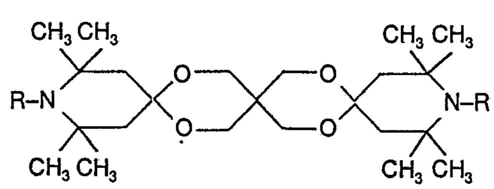

35 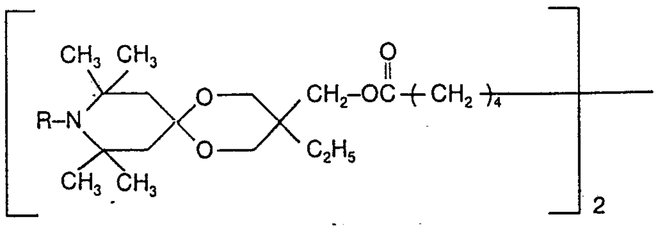

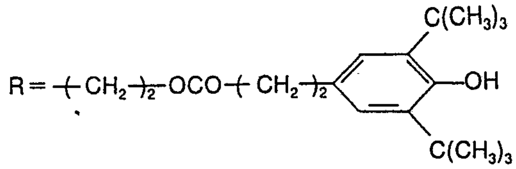

36 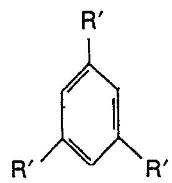

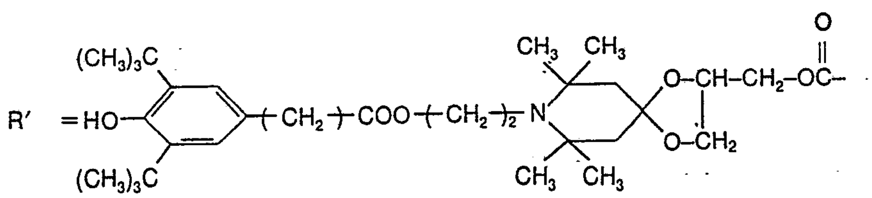

Die Stabilisatoren der Formel I oder II können allein oder zusammen mit anderen Verbindungen in bekannter Weise in ein photographisches Material eingearbeitet werden.

In der Regel werden die Stabilisatoren allein oder zusammen mit anderen Verbindungen, insbesondere mit Farbkupplern, in Form einer Dispersion in das photographische Material eingearbeitet, wobei diese Dispersion entweder kein Lösungsmittel oder hoch- oder tiefsiedende Lösungsmittel oder ein Gemisch solcher Lösungsmittel enthält. Eine weitere geeignete Einarbeitungsform besteht darin, dass man die Stabilisatoren allein oder zusammen mit weiteren Verbindungen zusammen mit einem Polymer in Form eines Latex in das photographische Material einarbeitet.

Die Dispersionen werden dann zur Herstellung der Schichten von farbphotographischen Aufzeichnungsmaterialien verwendet. Diese Schichten können z.B. Zwischen- oder Schutzschichten, insbesondere jedoch lichtempfindliche (blau-, grün- und rotempfindliche) Silberhalogenidemulsionsschichten sein, in denen bei der Entwicklung des belichteten Aufzeichnungsmaterials aus den entsprechenden Farbkupplern, die Blaugrün (Cyan)-, Purpur (Magenta)- und Gelbfarbstoffe gebildet werden.

Die Silberhalogenidschichten können beliebige Farbkuppler, insbesondere Blaugrün-, Purpur- und Gelb-Kuppler, die zur Bildung der genannten Farbstoffe und damit der Farbbilder verwendet werden, enthalten.

Da das Substrat die Wirkung und Stabilität der Verbindungen der Formel I oder II beeinflusst, werden Substrate (Lösungsmittel, Polymere) bevorzugt, die zusammen mit diesen Verbindungen eine möglichst gute Beständigkeit der zu stabilisierenden Verbindungen ergeben.

In der Regel werden die Stabilisatoren in Schichten eingearbeitet, die zusätzlich eine nach üblichen Methoden hergestellte und sensibilisierte Silberhalogenid-Dispersion enthalten. Sie können jedoch auch in zu Silberhalogenid enthaltenden Schichten benachbarten Schichten vorhanden sein.

Die erfindungsgemässen photographischen Materialien besitzen einen üblichen Aufbau und übliche Komponenten. Bevorzugt werden jedoch ein Aufbau und Komponenten, die die Wirksamkeit der Stabilisatoren der Formel I oder II verstärken oder zumindest nicht nachteilig beeinflussen.

Im photographischen Aufzeichnungsmaterial gemäss vorliegender Erfindung können die Stabilisatoren gemäss Formel I und II, ausser mit den Farbkupplern zusätzlich auch mit Ultraviolettabsorbern oder anderen Lichtschutzmitteln in der gleichen Schicht kombiniert werden.

Wenn die Diffusionstransfermethode angewandt wird, kann der Stabilisator auch in eine Empfangsschicht eingearbeitet werden.

Die erfindungsgemässen farbphotographischen Materialien können in bekannter Weise verarbeitet werden. Ferner können sie im Verlauf oder nach Verarbeitung in einer Weise behandelt werden, die ihre Stabilität weiter erhöht, beispielsweise durch die Behandlung in einem Stabilisatorbad oder das Aufbringen eines Schutzüberzugs.

Die erfindungsgemäss einzusetzenden Stabilisatoren eignen sich in gewissen Fällen auch zum Schutz farbphotographischer Schichten, in denen die Farbstoffe direkt in die Emulsion eingelagert werden und das Bild durch selektive Bleichung erzeugt wird.

Die Menge des Stabilisators oder der Stabilisatoren kann in weiten Grenzen schwanken und liegt etwa im Bereich von 1 bis 2000 mg, vorzugsweise 100 bis 800 und insbesondere 200–500 mg pro $m^2$ der Schicht, in die es (sie) eingearbeitet wird (werden).

Falls das photographische Material ein UV-Strahlung absorbierendes Mittel enthält, so kann dieses mit dem Stabilisator zusammen in einer Schicht oder auch in einer benachbarten Schicht vorhanden sein. Die Menge des UV-Absorbers oder der UV-Absorber kann in weiten Grenzen schwanken und liegt etwa im Bereich von 200 bis 2000 mg, vorzugsweise 400 bis 1000 mg pro $m^2$ der Schicht, in der er (sie) eingearbeitet wird (werden). Beispiele für Ultraviolett-Absorber sind Verbindungen vom Benzophenon-, Acrylnitril-, Thiazolidon-, Benztriazol-, Oxazol-, Thiazol- und Imidazoltyp.

Die mit dem erfindungsgemässen Aufzeichnungsmaterial durch Belichtung und Entwicklung erhaltenen Farbbilder zeigen eine sehr gute Lichtechtheit gegenüber sichtbarem und ultraviolettem Licht. Die Verbindungen der Formeln I und II sind praktisch farblos, so dass es zu keiner Verfärbung der Bilder kommt; ausserdem sind sie gut verträglich mit den üblichen, in den einzelnen Schichten vorhandenen photographischen Zusatzstoffen. Aufgrund ihrer guten Wirksamkeit kann man ihre Einsatzmenge herabsetzen und vermeidet so ihre Ausfällung oder ihr Auskristallisieren, wenn man sie als organische Lösung in die wässerigen Bindemittelemulsionen, die für die Herstellung photographischer Schichten verwendet werden, einarbeitet. Die einzelnen nach der Belichtung des photographischen Aufzeichnungsmaterials notwendigen Verarbeitungsschritte zur Herstellung der Farbbilder werden durch die Stabilisatoren nicht nachteilig beeinflusst. Ferner kann die bei blauempfindlichen Emulsionen häufig auftretende sogenannte Druckschleierbildung weitgehend zurückgedrängt werden. Diese kann z.B. auftreten, wenn auf photographische Materialien (Silberhalogenidemulsionsschichten, die sich auf einem Träger aus natürlichen oder synthetischen Materialien befinden) mechanische Beanspruchungen, z.B. Drehen, Biegen oder Reiben, während der Herstellung oder während der Behandlung vor der Entwicklung ausgeübt werden. (T.H. James, The Theory of Photographic Process 4. Auflage, Macmillan, New York, N.Y. 1977, Seite 23ff., S. 166ff.).

Anwendungsbeispiele

0,087 g des Gelbkupplers der Formel

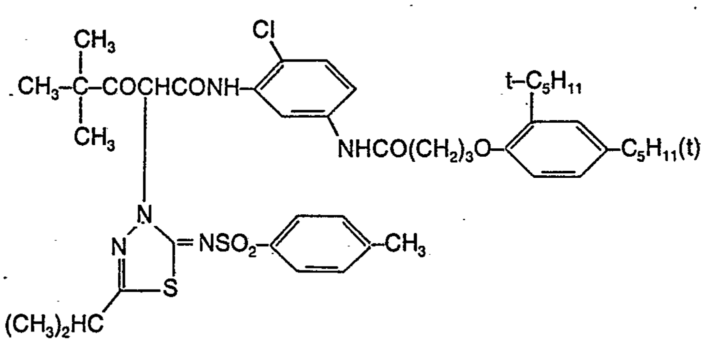

und 0,026 g eines der in den nachfolgenden Tabellen angegebenen Lichtschutzmittels werden in 2,0 ml eines Gemisches von Trikresylphosphat/Ethylacetat (1,5 g in 100 ml) gelöst. Zu dieser Lösung gibt man 7,0 ml einer 6%igen Gelatinelösung, 0,5 ml einer 8%igen Lösung des Netzmittels der Formel

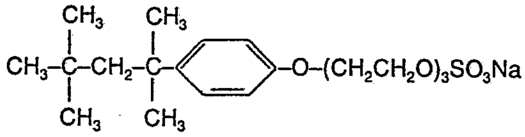

in Isopropanol/Wasser (3:4) und 0,5 ml Wasser und emulgiert mit Ultraschall bei einer Leistung von 100 Watt während 5 Minuten.

Zu 2,5 ml der so erhaltenen Emulsion gibt man 2,0 ml einer Silberbromid-Emulsion mit einem Silbergehalt von 6,0 g pro Liter, 0,7 ml einer 1%igen wässerigen Lösung des Härters der Formel

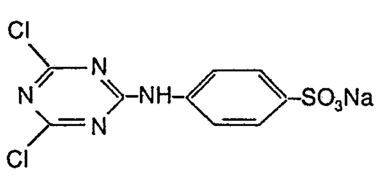

und 3,8 ml Wasser, stellt das Gemisch auf einen pH-Wert von 6,5 und vergiesst es auf ein auf eine Glasplatte aufgezogenes substriertes, kunststoffbeschichtetes, weisses Papier.

Nach dem Erstarren wird in einen Trockenschrank mit Umluft bei Raumtemperatur getrocknet.

Nach 7 Tagen werden auf 35 × 180 mm geschnittene Proben hinter einem Stufenkeil mit 3000 Lux · s belichtet und anschliessend im Ektaprint 2®-Prozess der Firma Kodak verarbeitet.

Die so erhaltenen Gelbkeile werden in einem Atlas Weather-Ometer mit einer 2500 W-Xenonlampe mit total 42 $kJoule/cm^2$ bestrahlt (eine Vergleichsprobe enthält kein Lichtschutzmittel).

Der dabei eingetretene Farbdichteverlust wird bestimmt durch Messung der Farbdichte bei $\lambda_{max}$ mit einem Densitometer (TR® 924 A der Fa. Macbeth).

Die Ergebnisse sind in der nachfolgenden Tabelle aufgeführt.

| Lichtschutzmittel Nr. | Dichteverlust aus Maximum in Prozent (Remission) |
|---|---|
| – | 36 |
| 8 | 15 |
| 9 | 15 |
| 16 | 16 |
| 20 | 15 |

**Patentansprüche**

1. Farbphotographisches Aufzeichnungsmaterial, das in mindestens einer lichtempfindlichen Silberhalogenidemulsionsschicht, einer Zwischenschicht und/oder einer Schutzschicht mindestens eine Polyalkylpiperidinverbindung als Stabilisator enthält, dadurch gekennzeichnet, dass die Polyalkylpiperidinverbindung einer der Formeln I oder II

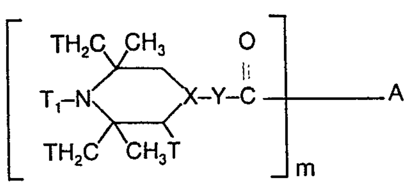 (I)

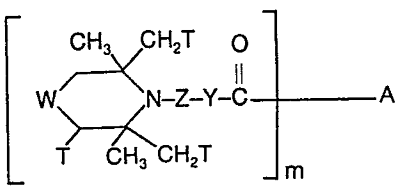 (II)

entspricht, worin

m die Zahlen 1 oder 2 bedeutet

A bei m = 1 eine Gruppe der Formel

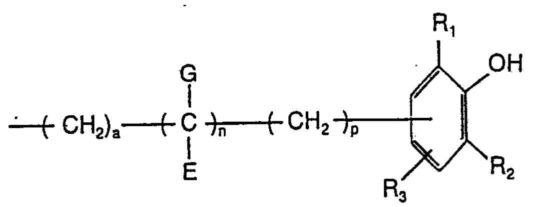

und bei m = 2 eine Gruppe der Formel

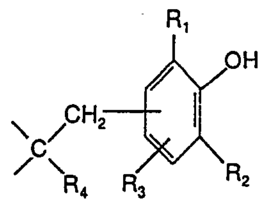

ist, worin

$R_1$ Wasserstoff, $C_1$–$C_{12}$ Alkyl, $C_5$–$C_8$ Cycloalkyl, $C_7$–$C_9$ Phenylalkyl, Phenyl oder $C_7$–$C_{10}$ Alkylphenyl bedeutet,

$R_2$ $C_1$–$C_8$ Alkyl, $C_5$–$C_8$ Cycloalkyl, $C_7$–$C_9$ Phenylalkyl, Phenyl oder $C_7$–$C_{10}$ Alkylphenyl ist,

$R_3$ Wasserstoff oder Methyl ist,

$R_4$ Wasserstoff, $C_1$–$C_{12}$ Alkyl, Allyl, Benzyl, Cyclohexyl oder eine Gruppe

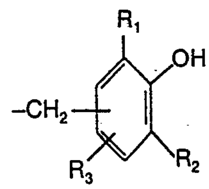 ist,

G Wasserstoff oder eine Gruppe

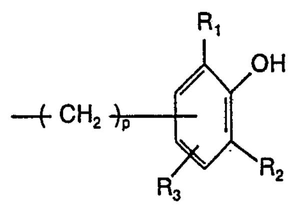

bedeutet,

E Wasserstoff, Methyl, –CN, $-COR_5$ oder $-COOR_5$ ist, wobei $R_5$ $C_1$–$C_8$ Alkyl oder $C_3$–$C_4$ Alkoxyalkyl bedeutet und

n und p unabhängig voneinander die Zahlen 0 oder 1 und a die Zahlen 0, 1 oder 2 bedeuten,

Y –O– oder $-N(R_6)-$ ist, wobei $R_6$ Wasserstoff, $C_1$–$C_{18}$ Alkyl, $C_3$–$C_{12}$ Alkenyl, $C_5$–$C_8$ Cycloalkyl, Phenyl, $C_7$–$C_{14}$ Alkaryl, $C_7$–$C_{14}$ Aralkyl oder $C_5$–$C_4$ Alkoxyalkyl ist

Z eine Gruppe $-{}^{*}CH_2-\underset{R_7}{\underset{|}{CH}}-(CH_2)_e-$,

worin $R_7$ Wasserstoff, Methyl, Ethyl, Phenoxymethyl, Phenyl oder $OR_8$ ist, $-OR_8$ aber nur dann, wenn e = 1 ist, und worin $R_8$ Wasserstoff oder –COL bedeutet, L $C_1$–$C_4$-Alkyl ist, e die Zahl 0 oder 1 ist und das *C am Piperidinstickstoff gebunden ist, bedeutet,

T Wasserstoff oder Methyl ist,

$T_1$ Hydroxy, $C_1$–$C_{12}$ Alkyl, $C_3$–$C_6$ Alkenylmethyl oder $C_3$–$C_4$ Alkinylmethyl, sowie $C_7$–$C_{14}$ Aralkyl, Glycidyl, durch Halogen, Cyano, $-COOR_9$ oder

$-CON(R_{10})(R_{11})$ substituiertes $C_1$–$C_4$-Alkyl, eine Gruppe $-COR_{12}$, $-COOR_9$ oder $-CON(R_{10})(R_{11})$, eine Gruppe $-CH_2-CH(R_{13})-OR_{14}$, $-SOR_{15}$, $-SO_2R_{15}$, $-OR_9$, $-OOCR_{12}$ ist, wobei $R_9$ $C_1$–$C_{12}$ Alkyl, Allyl, Cyclohexyl oder Benzyl, $R_{10}$ $C_1$–$C_{12}$ Alkyl, Allyl, Cyclohexyl, Benzyl, Phenyl oder $C_7$–$C_{10}$ Alkylphenyl, $R_{11}$ Wasserstoff, $C_1$–$C_{12}$-Alkyl, Allyl, Cyclohexyl oder Benzyl sind oder $R_{10}$ und $R_{11}$ zusammen mit dem N-Atom, an das sie gebunden sind, einen 5- oder 6-gliedrigen heterocyclischen Ring bilden, $R_{12}$ Wasserstoff, $C_1$–$C_{12}$ Alkyl, $C_2$–$C_6$ Alkenyl, Chlormethyl, $C_5$–$C_8$ Cycloalkyl, $C_7$–$C_{14}$ Aralkyl, Phenyl, $C_7$–$C_{10}$ Alkylphenyl oder durch 1 oder 2 $C_1$–$C_4$ Alkyl und 1 Hydroxyl substituiertes Phenyl, Phenylmethyl oder Phenylethyl, $R_{13}$ Wasserstoff, $C_1$–$C_4$ Alkyl, $C_3$–$C_4$ Alkoxyalkyl, Phenyl oder Phenoxymethyl, $R_{14}$ Wasserstoff, $C_1$–$C_{12}$ Alkyl, eine Gruppe $-COR_{12}$ oder $-CON(R_{10})(R_{11})$ und $R_{15}$ $C_1$–$C_{12}$ Alkyl, Phenyl oder $C_7$–$C_{10}$ Alkylphenyl bedeuten, oder

$T_1$ eine Gruppe der Formel

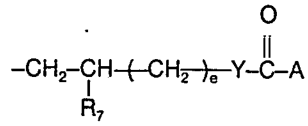

oder der Formel

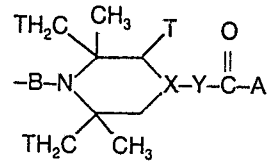

bedeutet, worin B eine Gruppe $C_rH_{2r}$ ist, in welcher r eine Zahl 2 bis 12 bedeutet, oder $C_4$–$C_8$ Alkenylen, $C_4$–$C_8$ Alkinylen, Phenylen, Xylylen, Bitolylen, $C_5$–$C_{12}$ Cycloalkylen oder eine Gruppe $-CONH-B_1-NHCO-$, worin $B_1$ eine Gruppe $C_rH_{2r}$, Phenylen, Naphthylen, Tolylen oder eine Gruppe der Formeln

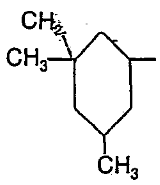 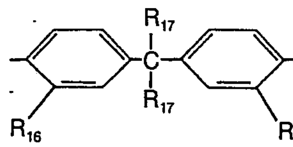 oder 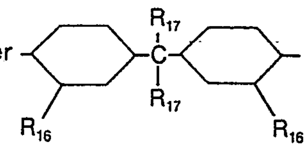

bedeutet, worin $R_{16}$ Wasserstoff oder Methyl, $R_{17}$ Wasserstoff, Methyl oder Ethyl sind,
X eine der Gruppen

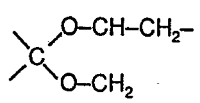 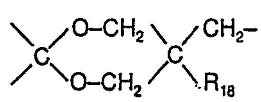

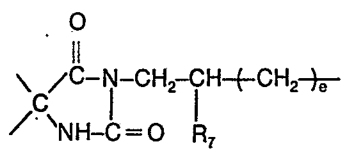

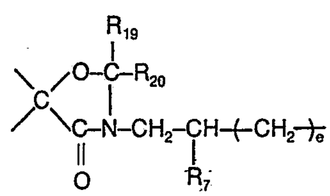 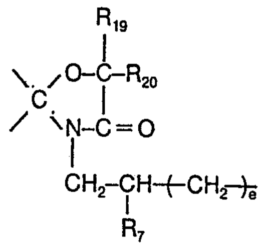

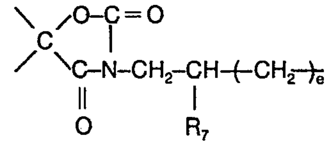 , und

W eine der Gruppen

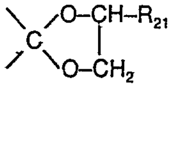 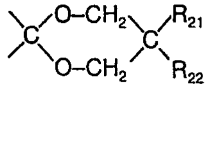 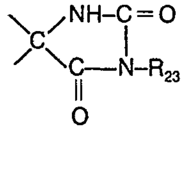

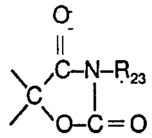

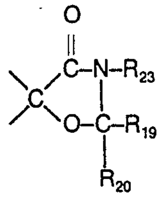

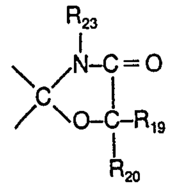

worin

$R_{18}$ Methyl oder Ethyl,

$R_{19}$ Wasserstoff, $C_1$–$C_{12}$ Alkyl, $C_5$–$C_8$ Cycloalkyl oder $C_7$–$C_{14}$ Aralkyl,

$R_{20}$ $C_1$–$C_{12}$ Alkyl, $C_5$–$C_8$ Cycloalkyl oder Phenyl sind oder

$R_{19}$ und $R_{20}$ zusammen mit dem C-Atom, an das sie gebunden sind, einen $C_5$–$C_{12}$ Cycloalkan- oder Alkylcycloalkanring bilden,

$R_{21}$ Wasserstoff, $C_1$–$C_{12}$ Alkyl, eine Gruppe der Formel $-CH_2-OCOR_{24}$ ist, worin $R_{24}$ Wasserstoff, $C_1$–$C_4$ Alkyl, $C_2$–$C_6$ Alkenyl, Cyclohexyl, Phenyl, Benzyl oder Chlormethyl oder eine Gruppe $-CH_2-O-SO_2R_{27}$, worin $R_{27}$ Methyl, Phenyl oder p-Tolyl ist, bedeutet, oder eine der Gruppen

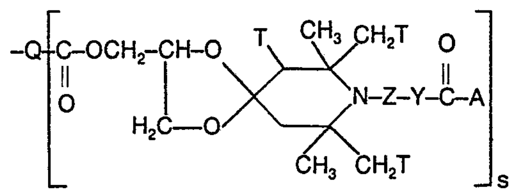

oder

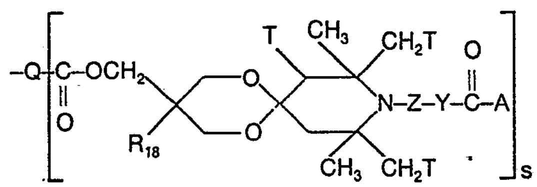

ist worin s die Zahl 1, 2 oder 3 bedeutet und

Q bei s = 1 die oben für B angegebene Bedeutung hat

bei s = 2 ein dreiwertiger Rest

der Formeln 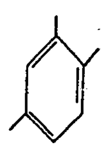 oder 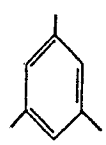

ist und

bei s = 3 ein vierwertiger Rest der Formeln

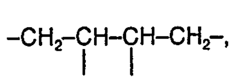, 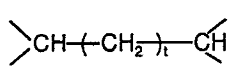

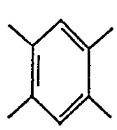 oder 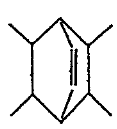

ist, wobei t

die Zahlen 1 bis 8 bedeutet, und

$R_{21}$ zusätzlich auch noch eine Gruppe der Formel $-CH_2O-SO\ R_{25}$, worin $R_{25}$ $C_1$–$C_4$ Alkyl, p-Tolyl oder Phenyl ist, eine Gruppe der Formel $-CH_2-CO-NHR_{26}$, worin $R_{26}$ Wasserstoff oder $C_1$–$C_4$ Alkyl ist,

$R_{22}$ Wasserstoff oder $C_1$–$C_4$ Alkyl und

$R_{23}$ Wasserstoff, $C_1$–$C_{12}$ Alkyl, $C_3$–$C_4$ Alkoxyalkyl, $C_5$–$C_8$ Cycloalkyl, Allyl oder Benzyl bedeuten, und, falls m = 1 ist, W zusätzlich auch eine Gruppe der Formel

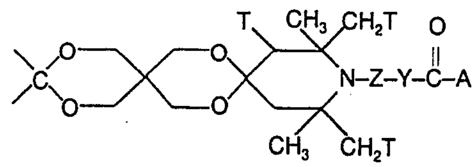

sein kann, wobei die wiederholt erwähnten Reste und Symbole immer die erstmals angegebene Bedeutung haben.

2. Farbphotographisches Aufzeichnungsmaterial gemäss Anspruch 1, dadurch gekennzeichnet, dass es als Stabilisator mindestens eine Polyalkylpiperidinverbindung enthält die einer der Formeln III oder IV

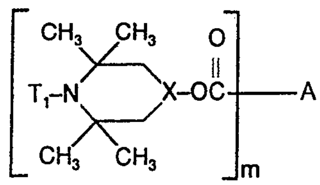 (III)

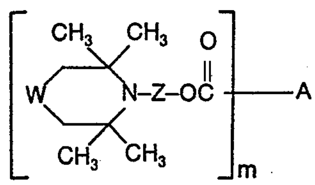 (IV)

entspricht, worin

m die Zahlen 1 oder 2 bedeutet

A bei m = 1 eine Gruppe der Formel

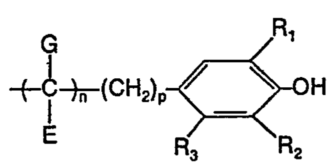

und bei m = 2 eine Gruppe der Formel

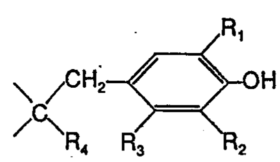

ist, worin

$R_1$ $C_1$–$C_4$ Alkyl,

$R_2$ Wasserstoff oder $C_1$–$C_4$ Alkyl,

$R_3$ Wasserstoff oder Methyl,

$R_4$ Wasserstoff, $C_1$–$C_4$ Alkyl oder eine Gruppe

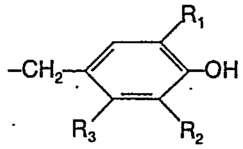

G Wasserstoff oder eine Gruppe

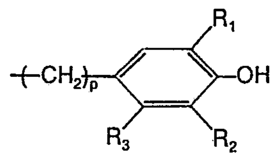

E Wasserstoff, Methyl, –CN oder –$COCH_3$ und

n und p unabhängig voneinander die Zahlen 0 oder 1 bedeuten,

Z eine Gruppe 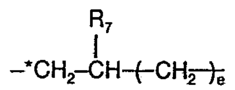 ist, worin

$R_7$ Wasserstoff, Methyl, Ethyl, Phenoxymethyl und Phenyl ist, e die Zahlen 0 oder 1 und das *C am Piperidinstickstoff gebunden ist,

$T_1$ Hydroxy, $C_1$–$C_4$ Alkoxy, Acetoxy, $C_1$–$C_4$ Alkyl, $C_3$–$C_4$ Alkenylmethyl, Propargyl, Glycidyl, Benzyl, durch –$COOR_9$ substituiertes Methyl oder Ethyl, eine Gruppe –$COR_{12}$, –$COOR_9$ oder –$CON(R_{10})(R_{11})$, eine Gruppe –$CH_2$–$CH(R_{13})$–$OR_{14}$ ist, wobei $R_9$ $C_1$–$C_8$ Alkyl, Allyl oder Cyclohexyl, $R_{10}$ $C_1$–$C_{12}$ Alkyl, Cyclohexyl oder Phenyl, $R_{11}$ Wasserstoff oder $C_1$–$C_{12}$ Alkyl sind oder $R_{10}$ und $R_{11}$ zusammen mit dem N-Atom, an das sie gebunden sind, einen 6-gliedrigen heterocyclischen Ring bilden, $R_{12}$ $C_1$–$C_{12}$ Alkyl, $C_2$–$C_4$ Alkenyl, Cyclohexyl, Benzyl, Phenyl oder 2-(3,5-Di-tert.-butyl-4-hydroxyphenyl)-ethyl, $R_{13}$ Wasserstoff, Methyl oder Phenyl und $R_{14}$ Wasserstoff, $C_1$–$C_4$ Alkyl, eine Gruppe –$COR_{12}$ oder –$CON(R_{10})(R_{11})$ bedeuten, oder

$T_1$ eine Gruppe der Formel

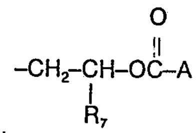

oder der Formel

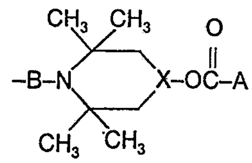

bedeutet, worin B eine Gruppe –(–$CH_2$–)$_r$– oder –CONH–(–$CH_2$–)$_r$–NHCO–, worin r die Zahlen 2 bis 8 bedeutet, oder $C_4$–$C_8$ Alkenylen, Xylylen oder Bitolylen ist,

X eine der Gruppen

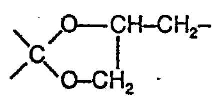

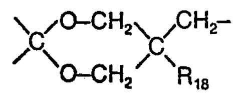

und

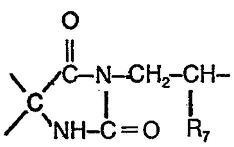

W eine der Gruppen

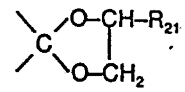

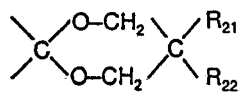

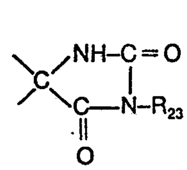

worin

$R_{18}$ Methyl oder Ethyl bedeutet

$R_{21}$ Wasserstoff-, $C_1$–$C_8$ Alkyl, eine Gruppe der Formel –$CH_2$–$OCOR_{24}$, worin $R_{24}$ $C_1$–$C_4$ Alkyl, Allyl, Phenyl oder Benzyl ist, oder eine Gruppe der Formel –$CH_2O$–$SO_2R_{27}$, worin $R_{27}$ Methyl, Phenyl oder p-Tolyl ist, bedeutet,

$R_{22}$ Wasserstoff, Methyl oder Ethyl ist und

$R_{23}$ Wasserstoff, $C_1$–$C_8$ Alkyl, Cyclohexyl, Allyl oder Benzyl bedeutet und, falls m = 1 ist, W zusätzlich auch eine Gruppe der Formel

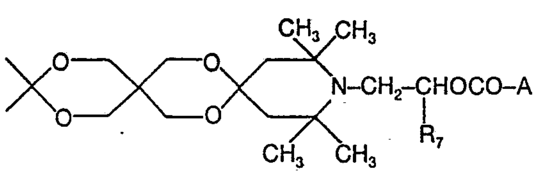

sein kann, wobei die in diesem Anspruch wiederholt erwähnten Reste und Symbole immer die in diesem Anspruch erstmals angegebene Bedeutung haben.

3. Farbphotographisches Aufzeichnungsmaterial gemäss Anspruch 1, dadurch gekennzeichnet, dass es als Stabilisator mindestens eine Polyalkylpiperidinverbindung der Formel III enthält, worin

m die Zahlen 1 oder 2 bedeutet,

A bei m = 1 eine Gruppe der Formel

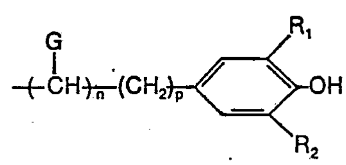

und bei m = 2 eine Gruppe der Formel

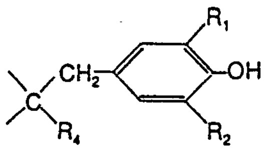

ist, worin

$R_1$ Wasserstoff, Methyl oder tert.-Butyl,

$R_2$ Methyl oder tert.-Butyl,

$R_4$ Wasserstoff, $C_1$–$C_4$ Alkyl oder eine Gruppe

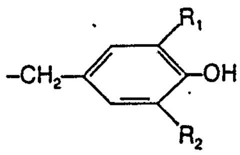

G Wasserstoff oder eine Gruppe

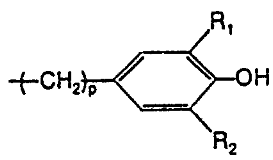

n und p unabhängig voneinander die Zahlen 0 oder 1 bedeuten,

$T_1$ Methoxy, Methyl, Allyl, Benzyl, Acetyl, Acryloyl oder eine Gruppe $-CON(R_{10})(R_{11})$ ist, wobei $R_{10}$ $C_1$–$C_4$ Alkyl, Cyclohexyl oder Phenyl $R_{11}$ Wasserstoff oder $C_1$–$C_4$ Alkyl bedeuten oder

$T_1$ eine Gruppe $-CH_2CH_2-OCO-A$ ist,

X eine der Gruppen

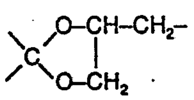

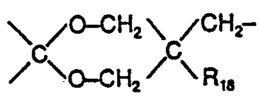

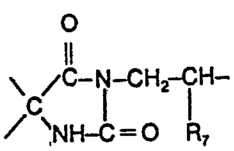

worin $R_7$ Wasserstoff, Methyl oder Phenyl und $R_{18}$ Methyl oder Ethyl ist, bedeutet, wobei die in diesem Anspruch wiederholt erwähnten Reste immer die in diesem Anspruch erstmals angegebene Bedeutung haben.

4. Farbphotographisches Aufzeichnungsmaterial gemäss Anspruch 1, dadurch gekennzeichnet, dass es als Stabilisator mindestens eine Polyalkylpiperidinverbindung der Formel IV enthält, worin

m die Zahlen 1 oder 2 bedeutet,

A bei m = 1 eine Gruppe der Formel

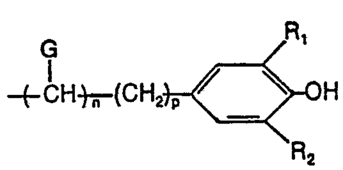

und bei m = 2 eine Gruppe der Formel

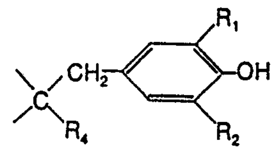

ist, worin

$R_1$ Wasserstoff, Methyl oder tert.-Butyl,

$R_2$ Methyl oder tert.-Butyl,

$R_4$ Wasserstoff, $C_1$–$C_4$ Alkyl oder eine Gruppe

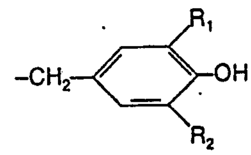

G Wasserstoff oder eine Gruppe

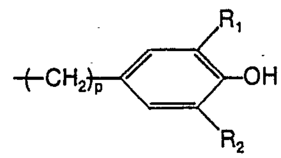

n und p unabhängig voneinander die Zahlen 0 oder 1 bedeuten,

Z eine Gruppe $-{}^*CH_2-\underset{R_7}{CH}-$

ist, worin $R_7$ Wasserstoff, Methyl oder Phenyl ist und das *C am Piperidinstickstoff gebunden ist,

$T_1$ Methoxy, Methyl, Allyl, Benzyl, Acetyl, Acryloyl oder eine Gruppe $-CON(R_{10})(R_{11})$, $-SOR_{15}$ oder $-SO_2R_{15}$ ist, wobei $R_{10}$ $C_1$–$C_4$ Alkyl, Cyclohexyl oder Phenyl, $R_{11}$ Wasserstoff oder $C_1$–$C_4$ Alkyl und $R_{15}$ Methyl, Phenyl oder p-Tolyl bedeuten oder

$T_1$ eine Gruppe $-CH_2CH_2-OCO-A$ ist

W eine der Gruppen

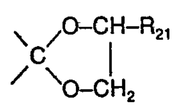

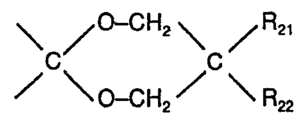

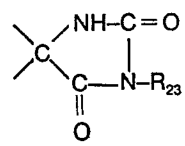

worin

$R_{21}$ Wasserstoff, $C_1$–$C_8$ Alkyl, eine Gruppe der Formel $-CH_2-OCOR_{24}$, worin $R_{24}$ $C_1$–$C_4$ Alkyl, Allyl oder Benzyl ist oder eine Gruppe der Formel $-CH_2O-SO_2R_{27}$, worin $R_{27}$ Methyl, Phenyl oder p-Tolyl ist, bedeutet,

$R_{22}$ Wasserstoff, Methyl oder Ethyl ist und

$R_{23}$ Wasserstoff, $C_1$–$C_8$ Alkyl, Allyl oder Benzyl bedeutet, und, falls m = 1 ist, W zusätzlich auch eine Gruppe der Formel

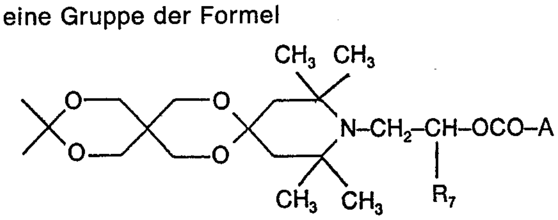

sein kann, wobei die in diesem Anspruch wiederholt erwähnten Reste immer die in diesem Anspruch erstmals angegebene Bedeutung haben.

5. Farbphotographisches Aufzeichnungsmaterial gemäss Anspruch 1, dadurch gekennzeichnet, dass es als Stabilisator mindestens eine Polyalkylpiperidinverbindung der Formel V enthält

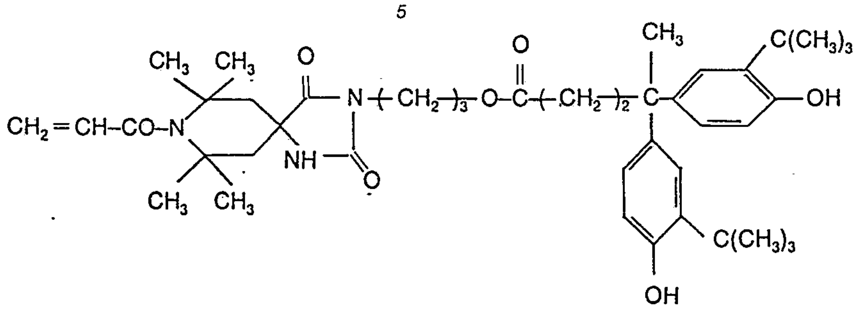

(V)

6. Farbphotographisches Aufzeichnungsmaterial gemäss Anspruch 1, dadurch gekennzeichnet, dass es als Stabilisator mindestens eine Polyalkylpiperidinverbindung der Formel VI enthält

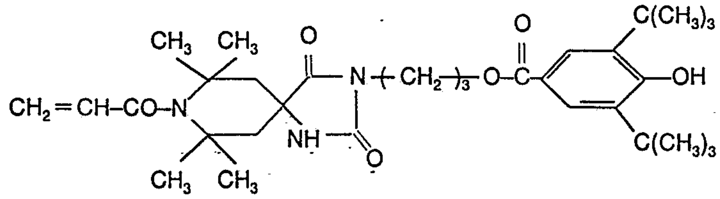

(VI)

7. Farbphotographisches Aufzeichnungsmaterial gemäss Anspruch 1, dadurch gekennzeichnet, dass es als Stabilisator mindestens eine Polyalkylpiperidinverbindung der Formel VII enthält

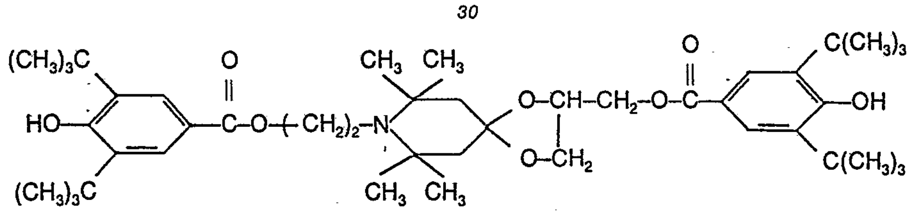

(VII).

8. Farbphotographisches Aufzeichnungsmaterial gemäss Anspruch 1, dadurch gekennzeichnet, dass es als Stabilisator mindestens eine Polyalkylpiperidinverbindung der Formel VIII enthält

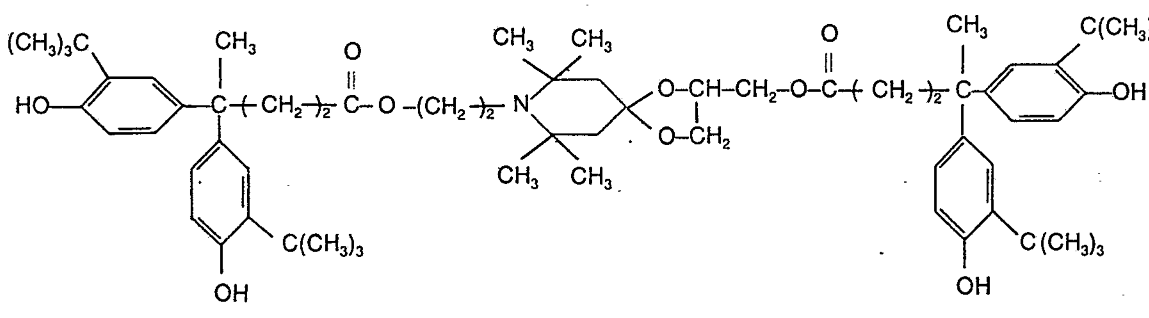

(VIII).

9. Farbphotographisches Aufzeichnungsmaterial gemäss Anspruch 1, dadurch gekennzeichnet, dass es die Stabilisatoren der Formel I oder II in Kombination mit Blaugrün-, Purpur- und Gelbkupplern enthält.

10. Farbphotographisches Aufzeichnungsmaterial gemäss Anspruch 1, dadurch gekennzeichnet, dass es die Stabilisatoren der Formeln I oder II in Kombination mit Ultraviolettabsorbern enthält.

11. Farbphotographisches Aufzeichnungsmaterial gemäss Anspruch 10, dadurch gekennzeichnet, dass die Ultraviolettabsorber Verbindungen von Benzophenon-, Acrylnitril-, Thiazolidon-, Benztriazol-, Oxazol-, Thiazol- oder Imidazoltyp sind.

12. Farbphotographisches Aufzeichnungsmaterial gemäss Anspruch 1, dadurch gekennzeichnet, dass es die Stabilisatoren der Formeln I oder II in Kombination mit Blaugrün-, Purpur- und Gelbkupplern und mit UV-Absorbern in der gleichen Schicht enthält.

13. Farbphotographisches Aufzeichnungsmaterial gemäss Anspruch 1, dadurch gekennzeichnet,

dass es 1 bis 2000 mg des Stabilisators pro $m^2$ der Schicht enthält, in die es eingearbeitet ist.

14. Verfahren zur Herstellung von photographischen Farbbildern durch bildmässige Belichtung und Farbentwicklung eines farbphotographischen Aufzeichnungsmaterials gemäss Anspruch 1.

## Claims

1. A colour-photographic recording material which, in at least one light-sensitive silver halide emulsion layer, one intermediate layer and/or one protective layer, contains, as a stabiliser, at least one polyalkylpiperidine compound, wherein the polyalkylpiperidine compound has one of the formulae I or II

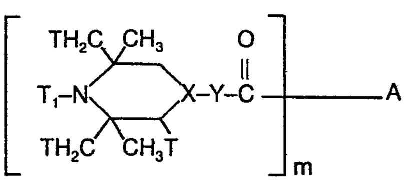 (I)

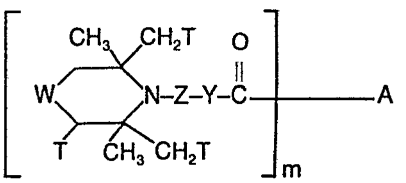 (II)

in which m is the number 1 or 2, if m = 1, A is a group of the formula

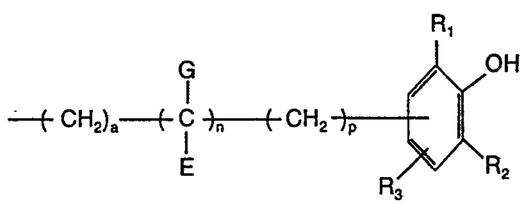

and, if m = 2, A is a group of the formula

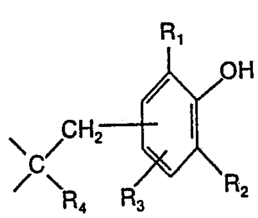

in which $R_1$ is hydrogen, $C_1$–$C_{12}$-alkyl, $C_5$–$C_8$-cycloalkyl, $C_7$–$C_9$-phenylalkyl, phenyl or $C_7$–$C_{10}$-alkylphenyl, $R_2$ is $C_1$–$C_8$-alkyl, $C_5$–$C_8$-cycloalkyl, $C_7$–$C_9$-phenylalkyl, phenyl or $C_7$–$C_{10}$-alkylphenyl, $R_3$ is hydrogen or methyl, $R_4$ is hydrogen, $C_1$–$C_{12}$-alkyl, allyl, benzyl, cyclohexyl or a group

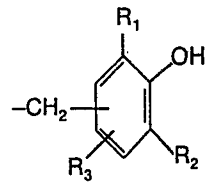

G is hydrogen or a group

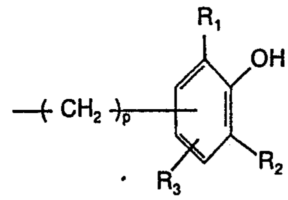

E is hydrogen, methyl, $-CN$, $-COR_5$ or $-COOR_5$, $R_5$ being $C_1$–$C_8$-alkyl or $C_3$–$C_4$-alkoxyalkyl, and n and p independently of one another are the numbers 0 or 1 and a is one of the numbers 0, 1 or 2, Y is $-O-$ or $-N(R_6)-$, $R_6$ being hydrogen, $C_1$–$C_{18}$-alkyl, $C_3$–$C_{12}$-alkenyl, $C_5$–$C_8$-cycloalkyl, phenyl, $C_7$–$C_{14}$-alkaryl, $C_7$–$C_{14}$-aralkyl or $C_3$–$C_4$ alkoxyalkyl, Z is a group 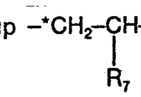,

in which $R_7$ is hydrogen, methyl, ethyl, phenoxymethyl, phenyl or $-OR_8$, but is only $-OR_8$ if e is 1, and in which $R_8$ is hydrogen or $-COL$, L is $C_1$–$C_4$-alkyl, e is the number 0 or 1 and the *C is attached to the piperidine nitrogen, T is hydrogen or methyl, $T_1$ is hydroxyl, $C_1$–$C_{12}$-alkyl, $C_3$–$C_6$-alkenylmethyl or $C_3$–$C_4$-alkynylmethyl and $C_7$–$C_{14}$-aralkyl, glycidyl, $C_1$–$C_4$-alkyl which is substituted by halogen, cyano, $-COOR_9$ or $-CON(R_{10})(R_{11})$, a group $-COR_{12}$, $-COOR_9$ or $-CON(R_{10})(R_{11})$ or a group $-CH_2-CH(R_{13})-OR_{14}$, $-SOR_{15}$, $-SO_2R_{15}$, $-OR_9$ or $-OOCR_{12}$ in which $R_9$ is $C_1$–$C_{12}$-alkyl, allyl, cyclohexyl or benzyl, $R_{10}$ is $C_1$–$C_{12}$-alkyl, allyl, cyclohexyl, benzyl, phenyl or $C_7$–$C_{10}$-alkylphenyl and $R_{11}$ is hydrogen, $C_1$–$C_{12}$-alkyl, allyl, cyclohexyl or benzyl, or $R_{10}$ and $R_{11}$, together with the N atom to which they are attached, form a 5-membered or 6-membered heterocyclic ring, $R_{12}$ is hydrogen, $C_1$–$C_{12}$-alkyl, $C_2$–$C_6$-alkenyl, chloromethyl, $C_5$–$C_8$-cycloalkyl, $C_7$–$C_{14}$-aralkyl, phenyl, $C_7$–$C_{10}$-alkylphenyl or phenyl, phenylmethyl or phenylethyl which are substituted by one or two $C_1$–$C_4$-alkyl groups and by one hydroxyl group, $R_{13}$ is hydrogen, $C_1$–$C_4$-alkyl, $C_3$–$C_4$-alkoxyalkyl, phenyl or phenoxymethyl, $R_{14}$ is hydrogen, $C_1$–$C_{12}$-alkyl or a group $-COR_{12}$ or $-CON(R_{10})(R_{11})$ and $R_{15}$ is $C_1$–$C_{12}$-alkyl, phenyl or $C_7$–$C_{10}$-alkylphenyl, or $T_1$ is a group of the formula

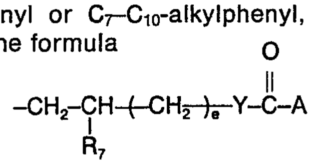

or of the formula

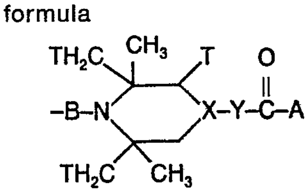

in which B is a group $C_rH_{2r}$ in which r is a number from 2 to 12, or is $C_4$–$C_8$-alkenylene, $C_4$–$C_8$-alkynylene, phenylene, xylylene, bitolylene, $C_5$–$C_{12}$-cycloalkylene or a group –CONH–$B_1$–NHCO– in which $B_1$ is a group $C_rH_{2r}$, phenylene, napthylene, tolylene or a group of the formulae

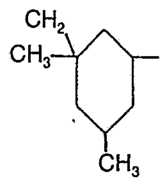 , 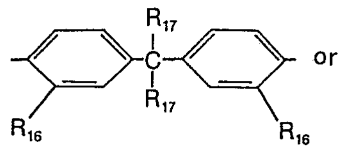 or 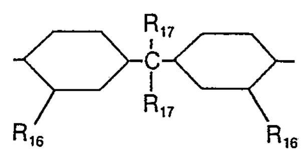

in which $R_{16}$ is hydrogen or methyl and $R_{17}$ is hydrogen, methyl or ethyl, X is one of the groups

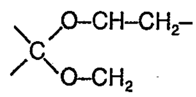

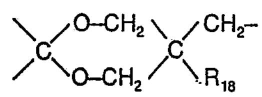

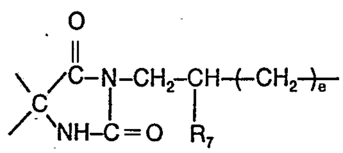

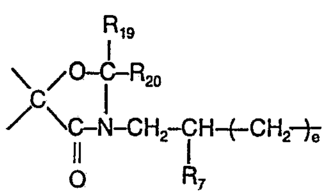

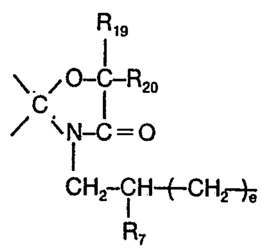

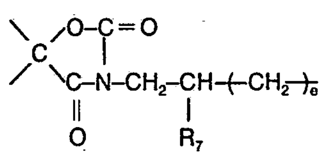

and W is one of the groups

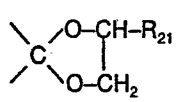

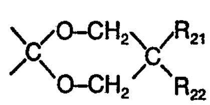

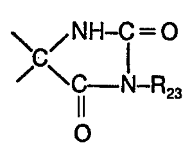

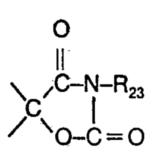

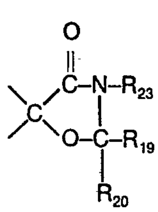

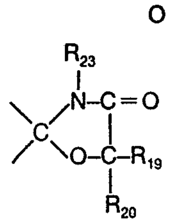

in which $R_{18}$ is methyl or ethyl, $R_{19}$ is hydrogen, $C_1$–$C_{12}$-alkyl, $C_5$–$C_8$-cycloalkyl or $C_7$–$C_{14}$-aralkyl, $R_{20}$ is $C_1$–$C_{12}$-alkyl, $C_5$–$C_8$-cycloalkyl or phenyl, or $R_{19}$ and $R_{20}$, together with the C atom to which they are attached, form a $C_5$–$C_{12}$ cycloalkane or alkylcycloalkane ring, $R_{21}$ is hydrogen, $C_1$–$C_{12}$-alkyl or a group of the formula –$CH_2$–$OCOR_{24}$ in which $R_{24}$ is hydrogen, $C_1$–$C_4$-alkyl, $C_2$–$C_6$-alkenyl, cyclohexyl, phenyl, benzyl or chloromethyl, or is a group –$CH_2$–O–$SO_2R_{27}$ in which $R_{27}$ is methyl, phenyl or p-tolyl, or is one of the groups

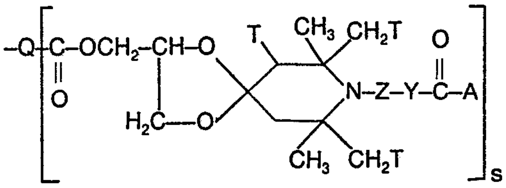

or

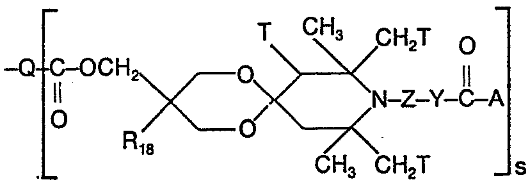

in which s is the number 1, 2 or 3, and, if s = 1, Q is as defined above for B, if s = 2, Q is a trivalent radical of the formulae

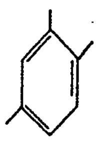 or 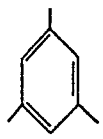

and, if s = 3, Q is a tetravalent radical of the formulae

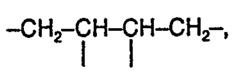 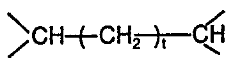

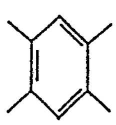 or 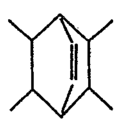

in which t is one of the numbers 1 to 8, and $R_{21}$ additionally is also a group of the formula $-CH_2O-S(O)_qR_{25}$ in which $R_{25}$ is $C_1-C_4$-alkyl, p-tolyl or phenyl and q is the number 1 or 2, or is a group of the formula $-CH_2O-NHR_{26}$ in which $R_{26}$ is hydrogen or $C_1-C_4$-alkyl, $R_{22}$ is hydrogen or $C_1-C_4$-alkyl and $R_{23}$ is hydrogen, $C_1-C_{12}$-alkyl, $C_3-C_4$-alkoxyalkyl, $C_5-C_8$-cycloalkyl, allyl or benzyl, and, if m is 1, W can additionally also be a group of the formula

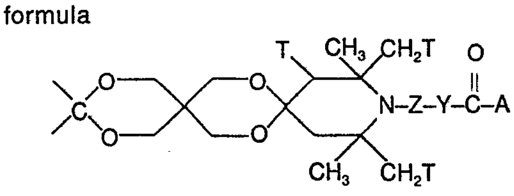

the radicals and symbols mentioned several times always being as defined initially.

2. A colour-photographic recording material according to Claim 1, which contains, as a stabiliser, at least one polyalkylpiperidine compound having one of the formulae III or IV

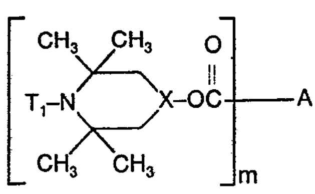

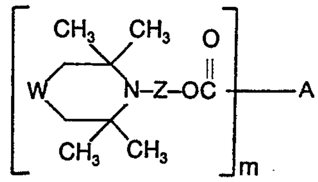

in which m is the number 1 or 2, if m = 1, A is a group of the formula

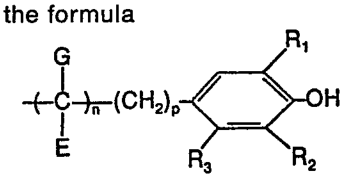

and, if m = 2, A is a group of the formula

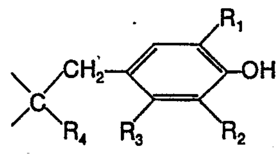

in which $R_1$ is $C_1-C_4$-alkyl, $R_2$ is hydrogen or $C_1-C_4$-alkyl, $R_3$ is hydrogen or methyl, $R_4$ is hydrogen, $C_1-C_4$-alkyl or a group

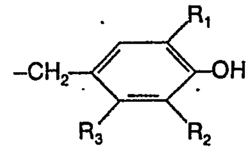

G is hydrogen or a group

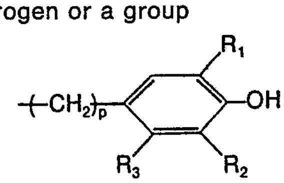

E is hydrogen, methyl, $-CN$ or $-COCH_3$ and n and p independently of one another are the number 0 or 1, Z is a group $-{}^*CH_2-\overset{R_7}{\overset{|}{C}}H-(-CH_2-)_e$ in which $R_7$ is hydrogen, methyl, ethyl, phenoxymethyl and phenyl, e is the number 0 or 1 and the *C is attached to the piperidine nitrogen, $T_1$ is hydroxyl, $C_1-C_4$-alkoxy, acetoxy, $C_1-C_4$-alkyl, $C_3-C_4$-alkenylmethyl, propargyl, glycidyl, benzyl, methyl or ethyl which is substituted by $-COOR_9$, a group $-COR_{12}$, $-COOR_9$ or $-CON(R_{10})(R_{11})$, or a group $-CH_2-CH(R_{13})-OR_{14}$ in which $R_9$ is $C_1-C_8$-alkyl, allyl or cyclohexyl, $R_{10}$ is $C_1-C_{12}$-alkyl, cyclohexyl or phenyl, $R_{11}$ is hydrogen or $C_1-C_{12}$-alkyl, or $R_{10}$ and $R_{11}$, together with the N atom to which they are attached, form a 6-membered heterocyclic ring, $R_{12}$ is $C_1-C_{12}$-alkyl, $C_2-C_4$-alkenyl, cyclohexyl, benzyl, phenyl or 2-(3,5-di-tert.-butyl-4-hydroxyphenyl)-ethyl, $R_{13}$ is hydrogen, methyl or phenyl and $R_{14}$ is hydrogen, $C_1-C_4$-alkyl or a group $-COR_{12}$ or $-CON(R_{10})(R_{11})$, or $T_1$ is a group of the formula

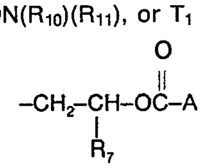

or of the formula

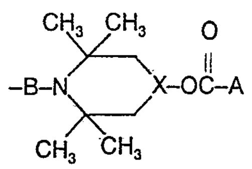

in which B is a group $-(CH_2)_r-$ or $-CONH-(CH_2)_r-NHCO-$ in which r is one of the numbers 2 to 8, or is $C_4$–$C_8$-alkenylene, xylylene or bitolylene, X is one of the groups

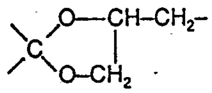

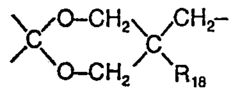

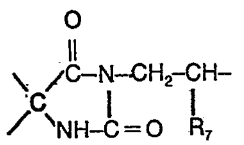

and W is one of the groups

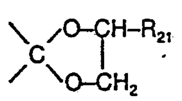

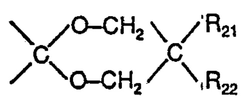

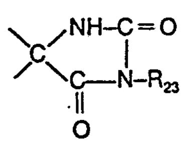

in which $R_{18}$ is methyl or ethyl, $R_{21}$ is hydrogen, $C_1$–$C_8$-alkyl, a group of the formula $-CH_2-OCOR_{24}$ in which $R_{24}$ is $C_1$–$C_4$-alkyl, allyl, phenyl or benzyl, or a group of the formula $-CH_2O-SO_2R_{27}$ in which $R_{27}$ is methyl, phenyl or p-tolyl, $R_{22}$ is hydrogen, methyl or ethyl and $R_{23}$ is hydrogen, $C_1$–$C_8$-alkyl, cyclohexyl, allyl or benzyl, and, if m i 1, W can additionally also be a group of the formula

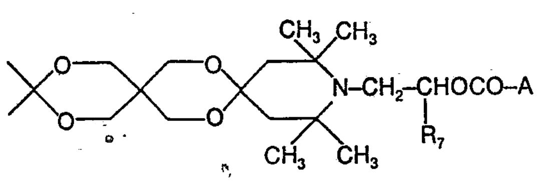

the radicals and symbols mentioned several times in this claim always being as definied initially in this claim.

3. A colour-photographic recording material according to Claim 1, which contains, as a stabiliser, at least one polyalkylpiperidine compound of the formula III in which m is the number 1 or 2, if m = 1, A is a group of the formula

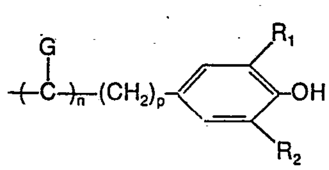

and, if m = 2, A is a group of the formula

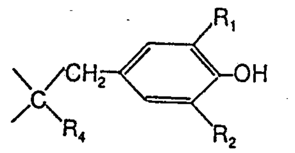

in which $R_1$ is hydrogen, methyl or tert.-butyl, $R_2$ is methyl or tert.-butyl, $R_4$ is hydrogen, $C_1$–$C_4$-alkyl or a group

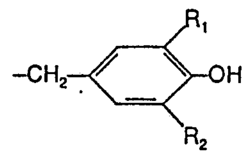

G is hydrogen or a group

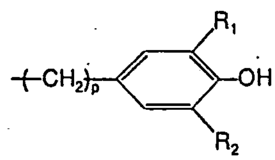

n and p independently of one another are the number 0 or 1, and $T_1$ is methoxy, methyl, allyl, benzyl, acetyl, acryloyl or a group $-CON(R_{10})(R_{11})$, $R_{10}$ being $C_1$–$C_4$-alkyl, cyclohexyl or phenyl and $R_{11}$ being hydrogen or $C_1$–$C_4$-alkyl, or $T_1$ is a group $-CH_2CH_2-OCO-A$, X is one of the groups

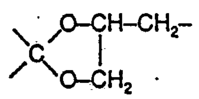

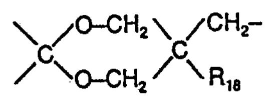

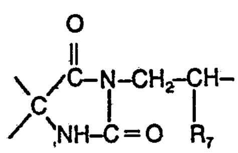

in which $R_7$ is hydrogen, methyl or phenyl and $R_{18}$ is methyl or ethyl, the radicals mentioned several times in this claim always being as defined initially in this claim.

4. A colour-photographic recording material according to Claim 1, which contains, as a stabiliser, at least one polyalkylpiperidine compound of the formula IV in which m is the number 1 or 2, if m = 1, A is a group of the formula

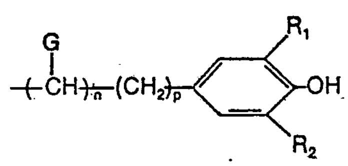

and, if m = 2, A is a group of the formula

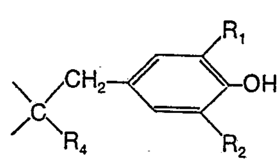

in which $R_1$ is hydrogen, methyl or tert.-butyl, $R_2$ is methyl or tert.-butyl, $R_4$ is hydrogen, $C_1$–$C_4$-alkyl or a group

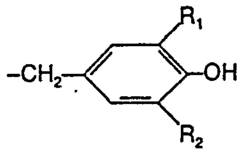

G is hydrogen or a group

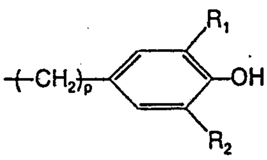

n and p independently of one another are the number 0 or 1, Z is a group 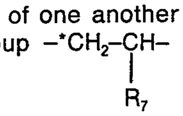

in which $R_7$ is hydrogen, methyl or phenyl and the *C is attached to the piperidine nitrogen, $T_1$ is methoxy, methyl, allyl, benzyl, acetyl, acryloyl or a group $-CON(R_{10})(R_{11})$, $-SOR_{15}$ or $-SO_2R_{15}$, $R_{10}$ being $C_1$–$C_4$-alkyl, cyclohexyl or phenyl, $R_{11}$ being hydrogen or $C_1$–$C_4$-alkyl and $R_{15}$ being methyl, phenyl or p-tolyl, or $T_1$ is a group $-CH_2CH_2-OCO-A$, W is one of the groups

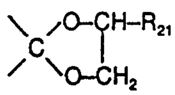

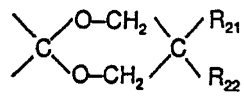

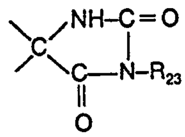

in which $R_{21}$ is hydrogen, $C_1$–$C_8$-alkyl, a group of the formula $-CH_2-OCOR_{24}$ in which $R_{24}$ is $C_1$–$C_4$-alkyl, allyl or benzyl, or a group of the formula $-CH_2O-SO_2R_{27}$ in which $R_{27}$ is methyl, phenyl or p-tolyl, $R_{22}$ is hydrogen, methyl or ethyl and $R_{23}$ is hydrogen, $C_1$–$C_8$-alkyl, allyl or benzyl, and, if m is 1, W can additionally also be a group of the formula

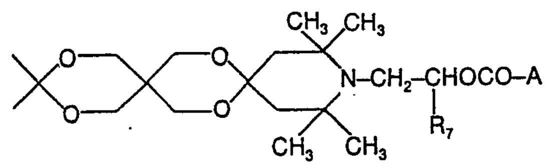

the radicals mentioned several times in this claim always being as defined initially in this claim.

5. A colour-photographic recording material according to Claim 1, which contains, as a stabiliser, at least one polyalkylpiperidine compound of the formula V

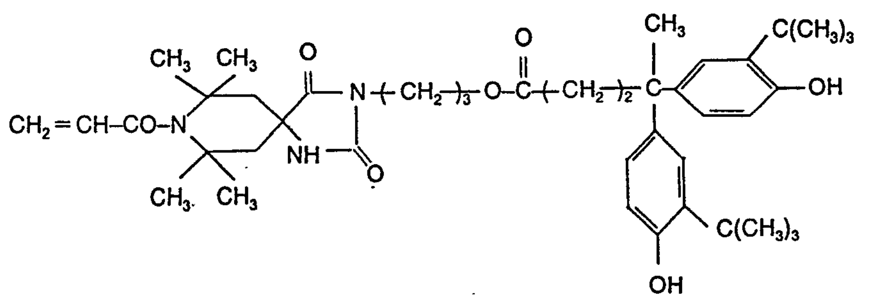

6. A colour-photographic recording material according to Claim 1, which contains, as a stabiliser, at least one polyalkylpiperidine compound of the formula VI

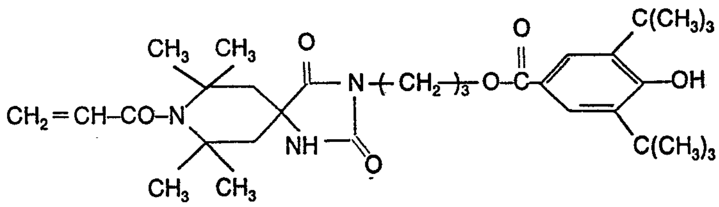

7. A colour-photographic recording material according to Claim 1, which contains, as a stabiliser, at least one polyalkylpiperidine compound of the formula VII

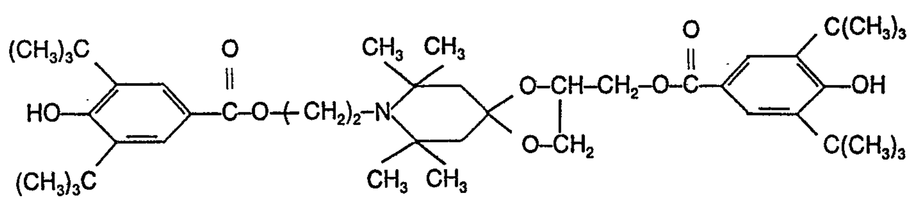

8. A colour-photographic recording material according to Claim 1, which contains, as a stabiliser, at least one polyalkylpiperidine compound of the formula VIII

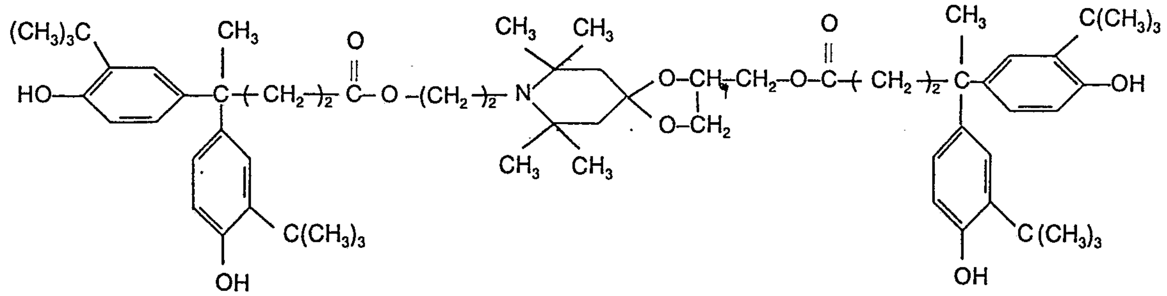

9. A colour-photographic recording material according to Claim 1, which contains the stabilisers of the formulae I or II in combination with cyan, magenta and yellow couplers.

10. A colour-photographic recording material according to Claim 1, which contains the stabilisers of the formulae I or II in combination with ultraviolet absorbers.

11. A colour-photographic recording material according to Claim 10, wherein the ultraviolet absorbers are compounds of the benzophenone, acrylonitrile, thiazolidone, benzotriazole, oxazole, thiazole or imidazole type.

12. A colour-photographic recording material according to Claim 1, which contains the stabilisers of the formulae I or II in combination with cyan, magenta and yellow couplers and with UV absorbers in the same layer.

13. A colour-photographic recording material according to Claim 1, which contains 1 to 2,000 mg of the stabiliser per $m^2$ of the layer into which it has been incorporated.

14. A process for the production of photographic colour images by image-wise exposure and colour development of a colour-photographic recording material according to Claim 1.

**Revendications**

1. Matière pour enregistrement photographique en couleurs qui contient un composé polyalkylpipéridinique, comme stabilisant, dans au moins une couche d'émulsion d'halogénure d'argent photosensible, une couche intermédiaire et/ou une couche protectrice, matière caractérisée en ce que le composé polyalky-pipéridinique répond à l'une des formules I et II:

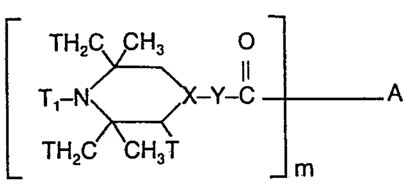

(I)

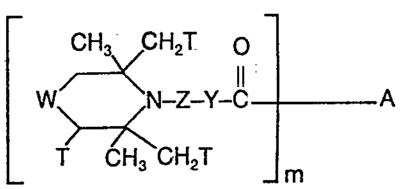

(II)

dans lesquelles:

m représente le nombre 1 ou le nombre 2,

A représente, dans le cas où m est égal à 1, un radical de formule:

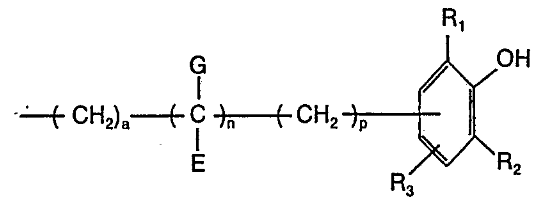

et, dans le cas où m est égal à 2, un radical de formule:

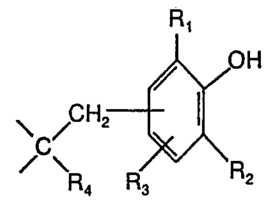

$R_1$ représente l'hydrogène, un alkyle en $C_1$–$C_{12}$, un cycloalkyle en $C_5$–$C_8$, un phénylalkyle en $C_7$–$C_9$, un phényle ou un alkylphényle en $C_7$–$C_{10}$,

$R_2$ représente un alkyle en $C_1$–$C_8$, un cycloalkyle en $C_5$–$C_8$, un phénylalkyle en $C_7$–$C_9$, un phényle ou un alkylphényle en $C_7$–$C_{10}$,

$R_3$ représente l'hydrogène ou un méthyle,

$R_4$ représente l'hydrogène, un alkyle en $C_1$–$C_{12}$, un allyle, un benzyle, un cyclohexyle ou un radical:

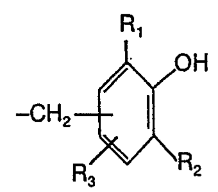

G représente l'hydrogène ou un radical

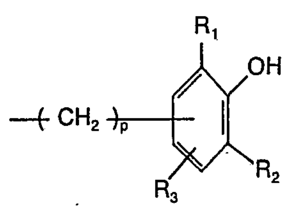,

E représente l'hydrogène, un méthyle, un radical –CN, ou l'un des radicaux $-COR_5$ et $-COOR_5$

dans lesquels $R_5$ représente un alkyle en $C_1$–$C_8$ ou un alcoxyalkyle en $C_3$ ou $C_4$,

n et p représentent chacun, indépendamment l'un de l'autre, le nombre 0 ou le nombre 1,

a représente l'un des nombres 0, 1 et 2,

Y représente –O– ou un radical $-N(R_6)-$ dans lequel $R_6$ représente l'hydrogène, un alkyle en $C_1$–$C_{18}$, un alcényle en $C_3$–$C_{12}$, un cycloalkyle en $C_5$–$C_8$, un phényle, un alkylaryle en $C_7$–$C_{14}$, un aralkyle en $C_7$–$C_{14}$ ou un alcoxyalkyle en $C_3$ ou $C_4$,

Z représente un radical 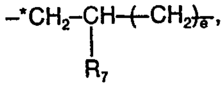

dans lequel l'atome de carbone *C est lié à l'azote pipéridinique, e représente le nombre 0 ou le nombre 1 et $R_7$ représente l'hydrogène, un méthyle, un éthyle, un phénoxyméthyle, un phényle ou, mais seulement dans le cas où e est égal à 1, un radical $-OR_8$ dans lequel $R_8$ représente l'hydrogène ou un radical –COL, le symbole L de ce dernier représentant un alkyle en $C_1$–$C_4$,

T représente l'hydrogène ou un méthyle,

$T_1$ représente un hydroxy, un alkyle en $C_1$–$C_{12}$, un alcénylméthyle en $C_3$–$C_6$, un alcynylméthyle en $C_3$ ou $C_4$, un aralkyle en $C_7$–$C_{14}$, un glycidyle, un alkyle en $C_1$–$C_4$ porteur d'un halogène, d'un cyano ou d'un radical $-COOR_9$ ou $-CON(R_{10})(R_{11})$, un radical $-COR_{12}$, $-COOR_9$ ou $-CON(R_{10})(R_{11})$, ou un radical $-CH_2-CH(R_{13})-OR_{14}$, $-SOR_{15}$, $-SO_2R_{15}$, $-OR_9$ ou $-OOCR_{12}$, dans lesquels $R_9$ représente un alkyle en $C_1$–$C_{12}$ un allyle, un cyclohexyle ou un benzyle, $R_{10}$ un alkyle en $C_1$–$C_{12}$, un allyle, un cyclohexyle, un benzyle, un phényle ou un alkylphényle en $C_7$–$C_{10}$, $R_{11}$ l'hydrogène, un alkyle en $C_1$–$C_{12}$, un allyle, un cyclohexyle ou un benzyle, ou encore $R_{10}$ et $R_{11}$ forment ensemble, et avec l'atome d'azote auquel ils sont liés, un hétérocycle à 5 ou 6 maillons, $R_{12}$ représente l'hydrogène, un alkyle en $C_1$–$C_{12}$, un alcényle en $C_2$–$C_6$, un chlorométhyle, un cycloalkyle en $C_5$–$C_8$, un aralkyle en $C_7$–$C_{14}$, un phényle, ou alkylphényle en $C_7$–$C_{10}$, ou un radical phényle, phénylméthyle ou phényléthyle portant un ou deux alkyles en $C_1$–$C_4$ et un hydroxy, $R_{13}$ l'hydrogène, un alkyle en $C_1$–$C_4$, un alcoxyalkyle en $C_3$ ou $C_4$, un phényle et/ou un phénoxyméthyle, $R_{14}$ l'hydrogène, un alkyle en $C_1$–$C_{12}$, ou un radical $-COR_{12}$ ou $-CON(R_{10})(R_{11})$, et $R_{15}$ un alkyle en $C_1$–$C_{12}$, un phényle ou un alkylphényle en $C_7$–$C_{10}$, ou

$T_1$ représente un radical répondant à l'une des formules

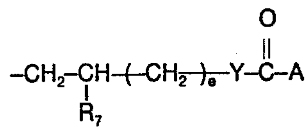

et

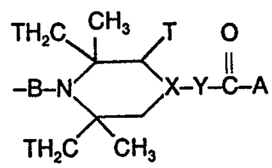

dans lesquelles B représente un radical $C_rH_{2r}$ dont l'indice r désigne un nombre de 2 à 12, ou représente un alcénylène en $C_4$–$C_8$, un alcynylène en $C_4$–$C_8$, un phénylène, un xylylène, un bitolylène, un cycloalkylène en $C_5$–$C_{12}$ ou un radical $-CONH-B_1-NHCO-$ dans lequel $B_1$ représente un radical $C_rH_{2r}$, un phénylène, un naphtylène, un tolylène ou un radical répondant à l'une des formules:

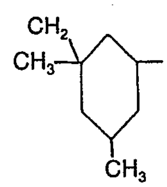 , 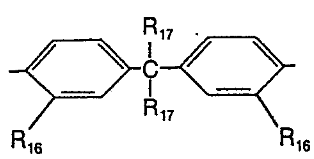 et 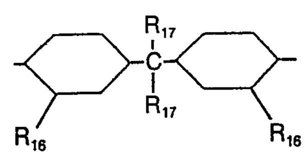

$R_{16}$ représente l'hydrogéne ou un méthyle,
$R_{17}$ représente l'hydrogène, un méthyle ou un éthyle,

X représente l'un des radicaux:

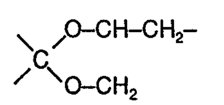

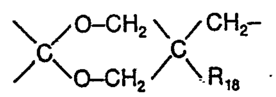

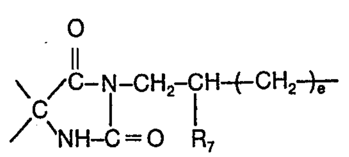

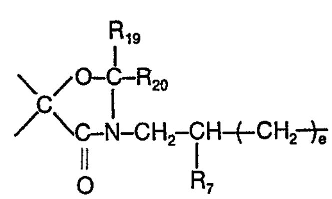

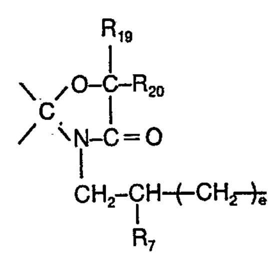

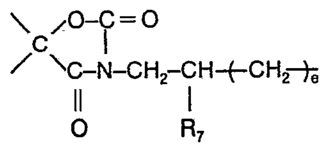

et

W représente l'un des radicaux:

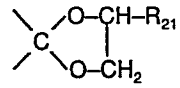

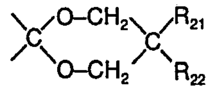

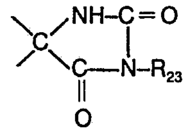

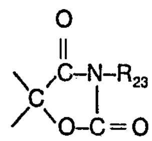

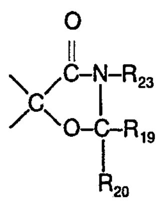

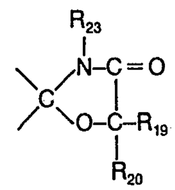

$R_{18}$ représente un méthyle ou un éthyle,

$R_{19}$ représente l'hydrogène, un alkyle en $C_1$–$C_{12}$, un cycloalkyle en $C_5$–$C_8$ ou un aralkyle en $C_7$–$C_{14}$,

$R_{20}$ représente un alkyle en $C_1$–$C_{12}$, un cycloalkyle en $C_5$–$C_8$ ou un phényle, ou

$R_{19}$ et $R_{20}$ forment ensemble, et avec l'atome de carbone ouquel ils sont liés, un noyau de cycloalcane ou d'alkylcycloalcane en $C_5$–$C_{12}$,

$R_{21}$ représente l'hydrogène, un alkyle en $C_1$–$C_{12}$, un radical $-CH_2-OCOR_{24}$ (dans lequel $R_{24}$ représente l'hydrogène, un alkyle en $C_1$–$C_4$ un alcényle en $C_2$–$C_6$, un cyclohexyle, un phényle, un benzyle ou un chlorométhyle), un radical $-CH_2-O-SO_2R_{27}$ (dans lequel $R_{27}$ représente un méthyle, un phényle ou un p-tolyle), ou un des radicaux:

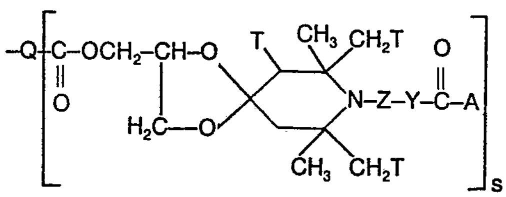

et

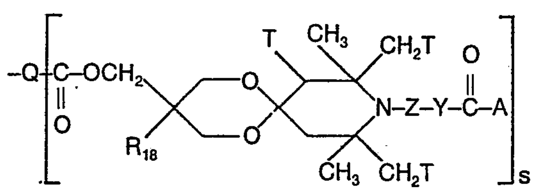

s représente l'un des nombres 1, 2 et 3,

Q représente, dans le cas où s est égal à 1, l'un des radicaux qui ont été mentionnés pour B, dans le cas où s est égal à 2, un radical trivalent répondant à l'une des formules:

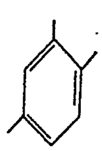

et

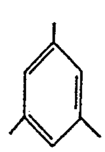

et dans le cas où s est égal à 3, un radical quadrivalent répondant à l'une des formules

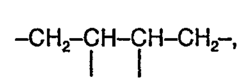

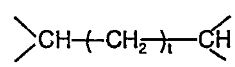

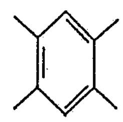

et

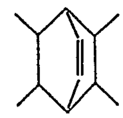

t représente un nombre de 1 à 8

$R_{21}$ peut en outre représenter un radical $-CH_2O-SO\ R_{25}$, dans lequel $R_{25}$ représente un alkyle en $C_1$–$C_4$, un p-tolyle ou un phényle, ou un radical $-CH_2-CO-NHR_{26}$ dans lequel $R_{26}$ représente l'hydrogène ou un alkyle en $C_1$–$C_4$,

$R_{22}$ représente l'hydrogène ou un alkyle en $C_1$–$C_4$,

$R_{23}$ représente l'hydrogène, un alkyle en $C_1$–$C_2$, un alcoxyalkyle en $C_3$ ou $C_4$, un cycloalkyle en $C_5$–$C_8$, un allyle ou un benzyle, et, dans le cas où m est égal à 1, W peut en outre représenter un radical de formule

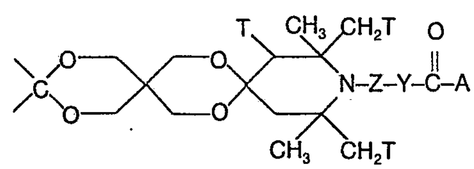

les radicaux et symboles mentionnés à plusieurs reprises ayant toujours la signification qui leur a été donnée la première fois.

2. Matière pour enregistrement photographique en couleurs selon la revendication 1, matière caractérisée en ce qu'elle contient, comme stabilisant, au moins un composé polyalkyl-pipéridinique répondant à l'une dès formules III et IV:

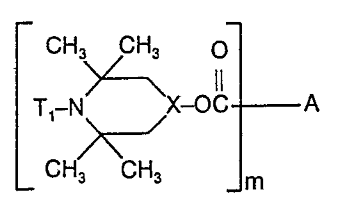

(III)

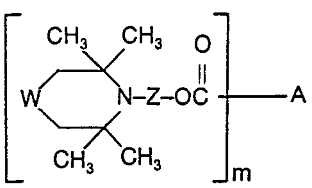

(IV)

dans lesquelles:

m représente le nombre 1 ou le nombre 2,

A représente, dans le cas où m est égal à 1, un radical de formule:

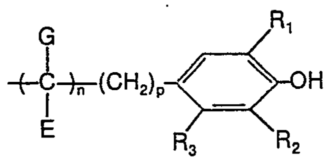

et, dans le cas, où m est égal à 2, un radical de formule:

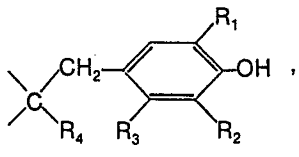

radicaux dans lesquels:

$R_1$ représente un alkyle en $C_1$–$C_4$,

$R_2$ représente l'hydrogène ou un alkyle en $C_1$–$C_4$,

$R_3$ représente l'hydrogène ou un méthyle,

$R_4$ représente l'hydrogène, un alkyle en $C_1$–$C_4$ ou un radical de formule:

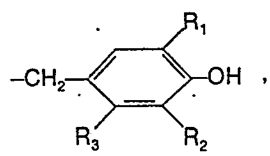

G représente l'hydrogène ou un radical:

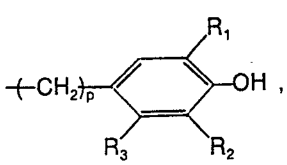

E représente l'hydrogène, un méthyle, –CN ou $-COCH_3$ et

n et p représentent chacun, indépendamment l'un de l'autre, le nombre 0 ou le nombre 1,

Z représente un radical: 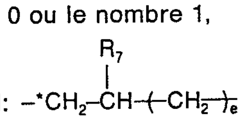

dans lequel $R_7$ représente l'hydrogène ou un radical méthyle, éthyle, phénoxyméthyle ou phényle, e représente le nombre 0 ou le nombre 1, et l'atome de carbone marqué d'un astérisque (*C) est lié à l'atome d'azote de la pipéridine,

$T_1$ représente un hydroxy, un alcoxy en $C_1$–$C_4$, un acétoxy, un alkyle en $C_1$–$C_4$, un alcénylméthyle en $C_3$ ou $C_4$, un propargyle, un glycidyle, un benzyle, un radical méthyle ou éthyle porteur d'un radical $-COOR_9$, ou l'un des radicaux $-COR_{12}$, $-COOR_9$, $-CON(R_{10})(R_{11})$ ou $-CH_2-CH(R_{13})-OR_{14}$, dans lesquels:

$R_9$ représente un alkyle en $C_1$–$C_8$, un allyle ou un cyclohexyle,

$R_{10}$ représente un alkyle en $C_1$–$C_{12}$, un cyclohexyle ou un phényle,

$R_{11}$ représente l'hydrogène ou un alkyle en $C_1$–$C_{12}$ ou

$R_{10}$ et $R_{11}$ forment ensemble, et avec l'atome d'azote auquel ils sont liés, un hétérocycle à 6 maillons,

$R_{12}$ représente un alkyle en $C_1$–$C_{12}$, un alcényle en $C_2$–$C_4$, un cyclohexyle, un benzyle, un phényle ou un (ditert-butyl-3,5 hydroxy-4 phényl)-2 éthyle,

$R_{13}$ représente l'hydrogène, un méthyle ou un phényle et

$R_{14}$ représente l'hydrogène, un alkyle en $C_1$–$C_4$, un radical $-COR_{12}$ ou un radical $-CON(R_{10})(R_{11})$, ou

$T_1$ représente un radical de formule:

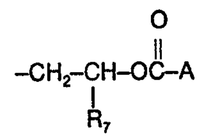

ou un radical de formule:

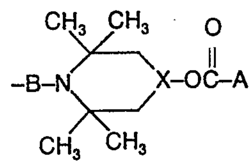

dans lesquels B représente un radical $-(-CH_2-)_r-$ ou $-CONH-(-CH_2-)_r-$ NHCO– dont l'indice r désigne un nombre de 2 à 8, ou représente un alkylène en $C_4$–$C_8$, un xylylène ou un bitolylène,

X représente l'un des radicaux:

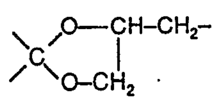

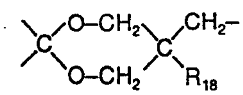

et

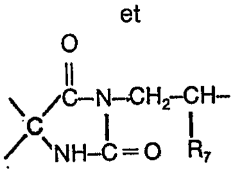

et

W représente l'un des radicaux:

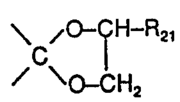

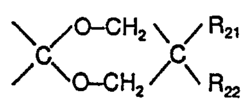

et

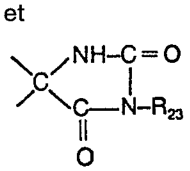

dans lesquels:

$R_{18}$ représente un méthyle ou un éthyle,

$R_{21}$ représente l'hydrogène, un alkyle en $C_1$–$C_8$,

un radical $-CH_2-OCOR_{24}$ dans lequel $R_{24}$ désigne un alkyle en $C_1-C_4$, un allyle, un phényle ou un benzyle, ou $R_{21}$ représente un radical $-CH_2O-SO_2R_{25}-$ dans lequel $R_{25}$ désigne un radical méthyle, phényle ou p-tolyle,

$R_{22}$ représente l'hydrogène, un méthyle ou un éthyle et

$R_{23}$ représente l'hydrogène, un alkyle en $C_1-C_8$, un cyclohexyle, un allyle ou un benzyle,

et, dans le cas où m est égal à 1, peut aussi représenter (il s'agit de W) un radical de formule:

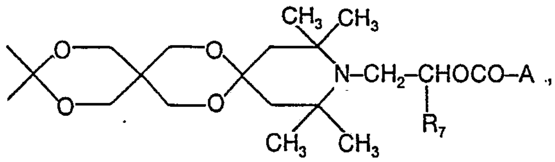

les radicaux et symboles mentionnés plusieurs fois dans cette revendication ayant toujours la signification qui leur a été donnée la première fois dans cette même revendication.

3. Matière pour enregistrement photographique en couleurs selon la revendication 1, matière caractérisée en ce qu'elle contient, comme stabilisant, au moins un composé polyalkyl-pipéridinique de formule III dans lequel:

m représente le nombre 1 ou le nombre 2,

A représente, dans le cas où m est égal à 1, un radical de formule:

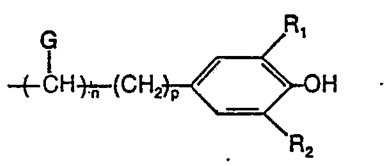

et, dans le cas où m est égal à 2, un radical de formule:

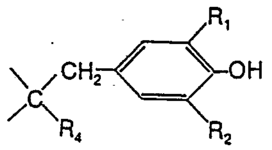

dans lesquels

$R_1$ représente l'hydrogène, un méthyle ou tert-butyle,

$R_2$ représente un méthyle ou un tert-butyle,

$R_4$ représente l'hydrogène, un alkyle en $C_1-C_4$ ou un radical:

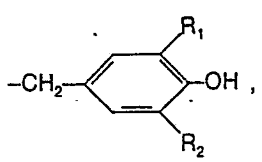

G représente l'hydrogène ou un radical:

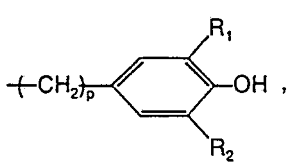

n et p représentent chacun, indépendamment l'un de l'autre, le nombre 0 ou le nombre 1,

$T_1$ représente un radical méthoxy, méthyle, allyle, benzyle, acétyle ou acryloyle, un radical $-CON(R_{10})(R_{11})$ dans lequel $R_{10}$ représente un alkyle en $C_1-C_4$, un cyclohexyle ou un phényle et $R_{11}$ l'hydrogène ou un alkyle en $C_1-C_4$, ou

$T_1$ représente un radical $-CH_2CH_2-OCO-A$,

X représente l'un des radicaux:

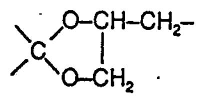

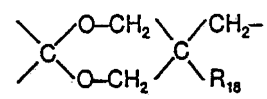

et

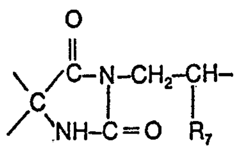

dans lesquels $R_7$ représente l'hydrogène, un méthyle ou un phényle et $R_{18}$ un méthyle ou un éthyle, les radicaux mentionnés plusieurs fois dans cette revendication ayant toujours la signification qui leur a été donnée la première fois dans cette revendication.

4. Matière pour enregistrement photographique en couleurs selon la revendication 1, matière caractérisée en ce qu'elle contient, comme stabilisant, au moins un composé polyalkyl-pipéridinique de formule IV dans lequel:

m représente le nombre 1 ou le nombre 2,

A représente, dans le cas où m est égal à 1, un radical de formule:

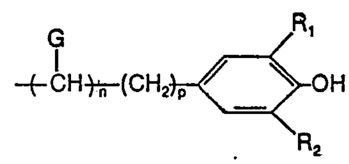

et, dans le cas où m est égal à 2, un radical de formule:

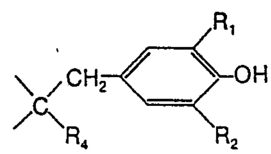

dans lesquels

$R_1$ représente l'hydrogène, un méthyle ou un tert-butyle

$R_2$ représente un méthyle ou un tert-butyle,

$R_4$ représente l'hydrogène, un alkyle en $C_1-C_4$ ou un radical:

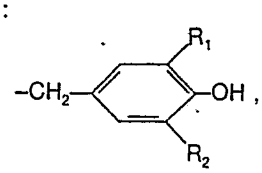

G représente l'hydrogène ou un radical:

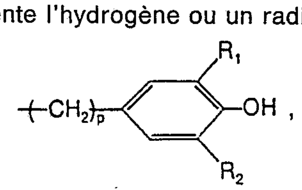

et

n et p représentent chacun, indépendamment l'un de l'autre, le nombre 0 ou le nombre 1,

Z représente un radical 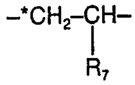

dans lequel $R_7$ représente l'hydrogène, un méthyle ou un phényle et l'atome de carbone *C est lié à l'atome d'azote pipéridinique,

$T_1$ représente un radical méthoxy, méthyle, allyle, benzyle, acétyle ou acryloyle, ou l'un des radicaux $-CON(R_{10})(R_{11})$, $-SOR_{15}$ et $-SO_2R_{15}$ dans lesquels $R_{10}$ représente un alkyle en $C_1$–$C_4$, un cyclohexyle ou un phényle, $R_{11}$ l'hydrogène ou un alkyle en $C_1$–$C_4$ et $R_{15}$ un méthyle, un phényle ou un p-tolyle, ou

$T_1$ représente un radical $-CH_2CH_2-OCO-A$,

W représente l'un des radicaux:

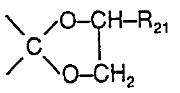

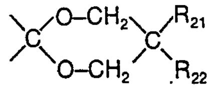

et

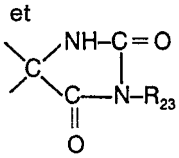

dans lesquels:

$R_{21}$ représente l'hydrogène, un alkyle en $C_1$–$C_8$, un radical $-CH_2-OCOR_{24}$ dans lequel $R_{24}$ désigne un alkyle en $C_1$–$C_4$, un allyle ou un benzyle, ou un radical $-CH_2O-SO_2R_{27}$ dans lequel $R_{27}$ représente un méthyle, un phényle ou un p-tolyle,

$R_{22}$ représente l'hydrogène, un méthyle ou un éthyle et

$R_{23}$ représente l'hydrogène, un alkyle en $C_1$–$C_8$, un allyle ou un benzyle,

et, dans le cas où m est égal à 1, W peut aussi représenter un radical de formule:

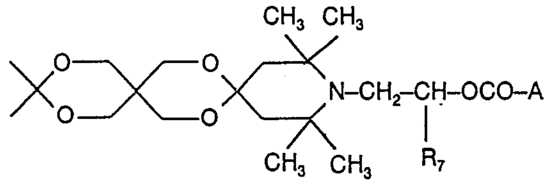

les radicaux mentionnés plusieurs fois dans cette revendication ayant toujours la signification qui leur a été donnée la première fois dans cette revendication.

5. Matière pour enregistrement photographique en couleurs selon la revendication 1, matière caractérisée en ce qu'elle contient, comme stabilisant, au moins un composé polyalkyl-pipéridinique de formule V:

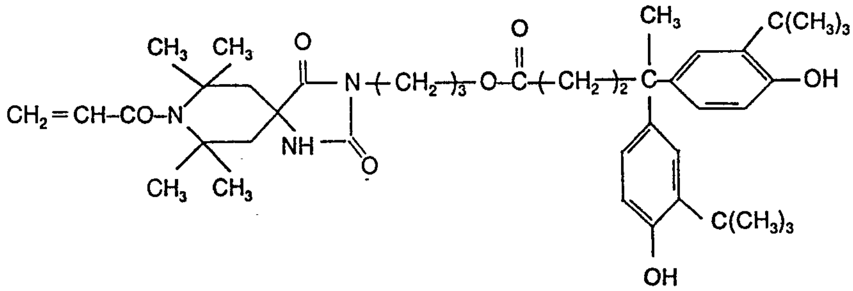

(V) .

6. Matière pour enregistrement photographique en couleurs selon la revendication 1, matière caractérisée en ce qu'elle contient, comme stabilisant, au moins un composé polyalkyl-pipéridinique de formule VI:

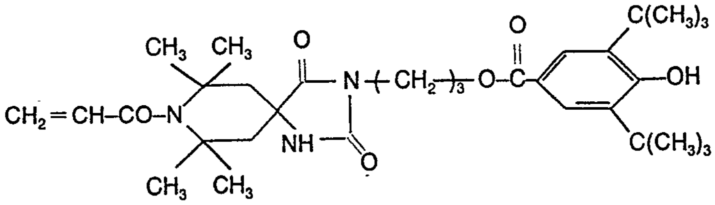

(VI) ,

7. Matière pour enregistrement photographique en couleurs selon la revendication 1, matière caractérisée en ce qu'elle contient, comme stabilisant, au moins un composé polyalkyl-pipéridinique de formule VII:

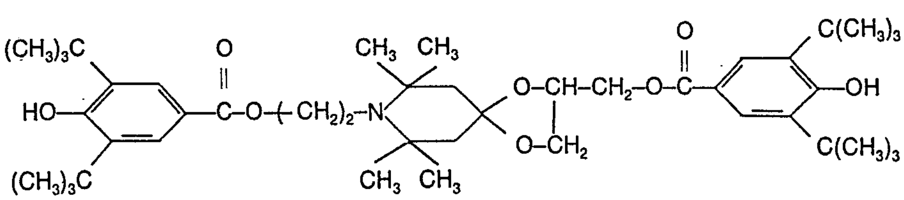

(VII).

8. Matière pour enregistrement photographique en couleurs selon la revendication 1, matière caractérisée en ce qu'elle contient, comme stabilisant, au moins un composé polyalkyl-pipéridinique de formule VIII:

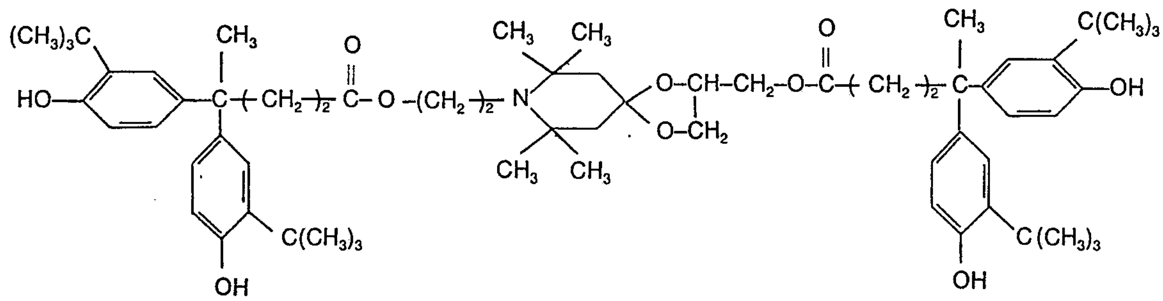

(VIII).

9. Matière pour enregistrement photographique en couleurs selon la revendication 1, matière qui contient des stabilisants de formule I ou II en association avec des copulants turquoise, pourpre et jaune.

10. Matière pour enregistrement photographique en couleurs selon la revendication 1, matière caractérisée en ce qu'elle contient des stabilisants de formule I ou II en association avec des absorbeurs de rayons ultraviolets.

11. Matière pour enregistrement photographique en couleurs selon la revendication 10, caractérisée en ce que les absorbeurs de rayons ultraviolets sont des composés du type des benzophénones, des acrylonitriles, des thiazolidones, des benzotriazoles, des oxazoles, des thiazoles ou des imidazoles.

12. Matière pour enregistrement photographique en couleurs selon la revendication 1, caractérisée en ce qu'elle contient, dans la même couche, des stabilisants de formule I ou II en association avec des copulants turquoise, pourpre et jaune et avec des absorbeurs de rayons ultraviolets.

13. Matière pour enregistrement photographique en couleurs selon la revendication 1, caractérisée en ce qu'elle contient de 1 à 2000 mg du stabilisant par mètre carré de la couche au sein de laquelle il est incorporé.

14. Procédé pour réaliser des images photographiques en couleurs, procédé selon lequel on expose à la lumière, conformément à une image, une matière pour enregistrement photographique en couleurs selon la revendication 1 et on développe les couleurs.